# EUROPEAN PATENT APPLICATION

(11) **EP 3 213 702 A1**
(43) Date of publication of application: **06.09.2017**
(21) Application number: 15853992.4
(22) Date of filing: 28.10.2015
(51) Int. Cl.: A61B 17/32, A61B 18/12

(54) **ENDOSCOPE SCISSORS AND ENDOSCOPIC HIGH-FREQUENCY TREATMENT TOOL**

(30) Priority: 29.10.2014 JP 2014219976; 30.10.2014 JP 2014221892; 19.10.2015 JP 2015205864; 19.10.2015 JP 2015205865
(71) Applicant: Sumitomo Bakelite Co., Ltd., Shinagawa-ku Tokyo 140-0002 (JP)
(72) Inventor: ISHII, Yasuhisa, Akita-shi, Akita 011-8510 (JP)
(74) Representative: Müller-Boré & Partner Patentanwälte PartG mbB
(86) International application number: PCT/JP2015/080429
(87) International publication number: WO 2016/068204

(57) **Abstract**

According to the present invention, there are provided endoscopic scissors, in which scissor pieces can precisely incise target biological tissue without slipping. For example, a minimally invasive technique can be performed without an excessively deep incision in which the incision reaches a muscle layer. The endoscopic scissors of the present invention are scissors (100) for an endoscope, which are used by being inserted into a forceps hole of the endoscope and incise biological tissue. The scissors (100) for an endoscope include a pair of scissor pieces (12) and (22) that face and overlap each other and are pivotally supported by a turning pivot (29) extending in an overlapping direction so as to be able to be opened and closed, and an operation wire (30). The pair of scissor pieces (12) and (22) individually has cutting portions (13) and (23) gripping and incising biological tissue. At tip end portions of at least one of the pair of scissor pieces (12) and (22), a claw portion (14) gripping biological tissue is formed so as to protrude in a closing direction beyond the cutting portion (13). Concave portions (15) and (25) penetrating the cutting portions (13) and (23) in a thickness direction are locally formed in the cutting portions (13) and (23) of the scissor pieces (12) and (22), and the cutting portions (13) and (23) excluding the claw portion (14) and the concave portions (15) and (25) are formed so as to have flat and linear shapes extending in a tip end - base end direction.

## Description

### Technical Field

The present invention relates to endoscopic scissors, which are used by being inserted into a forceps hole of the endoscope, and an endoscopic high frequency treatment tool.

Priority is claimed on Japanese Patent Application No. 2014-219976, filed on October 29, 2014, Japanese Patent Application No. 2014-221892, filed on October 30, 2014, Japanese Patent Application No. 2015-205864, filed on October 19, 2015, and Japanese Patent Application No. 2015-205865, filed on October 19, 2015, the contents of which are incorporated herein by reference.

### Background Art

Due to the minimal invasiveness thereof, endoscopic surgery is adopted in a surgical operation or the like in which biological tissue, for example, a lesion tissue is excised. As such types of endoscopic treatment tools, an instrument which ligates biological tissue with an annular snare and excises the biological tissue, gripping forceps having cup-shaped or plate-shaped forcep pieces, endoscopic scissors incising biological tissue with scissor pieces, and the like are known.

PTL 1 discloses gripping forceps performing an opening-closing operation of a pair of plate-shaped forcep pieces in which serrated crocodile teeth are entirely formed. At a tip end portion of the forcep piece, a claw portion protruding in a closing direction is formed, and a plurality of the crocodile teeth are provided in the forcep piece substantially in its entirety so as to be arranged in a tip end - base end direction while being concatenated with the claw portion.

The gripping forceps are instruments which strongly grip biological tissue and perform an excision such that the biological tissue is gouged out. That is, as illustrated in FIG. 7 of PTL 1, the gripping forceps are instruments which grip a region having a predetermined extent including an excision object site such as lesion tissue and gouge out the entire region. In contrast, the endoscopic scissors are instruments which apply a shearing force to biological tissue with a pair of scissor pieces overlapping each other and grip the biological tissue. As necessary, the endoscopic scissors apply a high frequency voltage to biological tissue such that the biological tissue is cauterized and incised. The endoscopic scissors are distinguished from the gripping forceps. The endoscopic scissors are instruments which excise an excision object site by circumferentially incising biological tissue along the periphery of the excision object site such as a lesion tissue. Since the endoscopic scissors can precisely excise only a target site in the biological tissue, it is possible to perform a minimally invasive technique which is further improved compared to the gripping forceps.

PTL 2 discloses an example of the endoscopic scissors. At a tip end portion of a scissor piece in the endoscopic scissors, a claw portion protruding in a closing direction is formed, and in a state where biological tissue is gripped, the biological tissue can be precisely incised by the scissor pieces. In FIG. 8 of PTL 2, it is disclosed that cutting portions of the scissor pieces are formed in a serrated manner. When the cutting portions are formed in the serrated manner and are bent upward and downward, comparatively hard biological tissue can also be incised. More specifically, in the scissor piece of PTL 2, a plurality of serrated teeth are formed in the cutting portion substantially in its entirety in the tip end - base end direction while being concatenated with the claw portion at the tip end. In other words, the claw portion protrudes so as to rise from a concave portion of the serrated teeth at the tip end.

PTL 3 discloses another example of the endoscopic scissors. In a cutting portion (cutting face) of a scissor piece (blade) of the endoscopic scissors, a recess is formed. Such a recess is formed shallow so as not to penetrate the scissor piece in the thickness direction, and the recess functions as a slip-resistant member when biological tissue is incised.

In addition, in the related art, as medical instruments for executing treatment such as an excision of a lesion site or the like inside a body cavity, an incision of biological tissue, and the like, there is a known endoscopic high frequency treatment tool, which is used together with an endoscope and in which a pair of gripping pieces is disposed at the tip end portion of an operation wire (operation wire).

For example, PTL 4 discloses an endoscopic high frequency treatment tool (hereinafter, will also be referred to as "technology 1 in the related art") including a pair of scissor pieces which are mutually and pivotally supported by a support pin and are turnably displaced between a released state and a closed state. The scissor pieces are displaced between a released state and a closed state due to an advance/retreat operation performed through the operation wire. The technology 1 in the related art includes a connection terminal for applying high frequency power source to cutting portions provided in the scissor pieces.

The scissor pieces of the technology 1 in the related art have the conductive cutting portions and cauterize biological tissue by interposing the biological tissue with the pair of scissor pieces and applying a high frequency voltage to the cutting portions. Meanwhile, on the outer surfaces of the scissor pieces excluding the cutting portions and the outer surface of a link mechanism interlocked with the scissor pieces, insulation coats are formed. Accordingly, in the technology 1 in the related art, when high frequency power source is applied, biological tissue is prevented from being in contact with places other than the cutting portions, and cauterization of unscheduled places is prevented.

Here, as illustrated in FIGS. 4 and 6 of PTL 4, the technology 1 in the related art includes a tip end support frame (hereinafter, will be referred to as holding frame) having a slit in the longitudinal direction. The pair of scissor pieces of the technology 1 in the related art and the link mechanism interlocked with the pair of scissor pieces are disposed within the slit and are supported by the holding frame. The pair of scissor pieces abuts on inner surfaces (wall surfaces configuring the slit) of the holding frame, and thus, disposition positions thereof are stabilized. The pair of scissor pieces is turnably and pivotally supported by the support pin which is held across between the wall surfaces which configure the slit and face each other. Accordingly, the pair of scissor pieces is disposed in the slit without deviating in an axial direction of the support pin and in a direction perpendicular to the axial direction.

### Citation List

### Patent Literature

[PTL 1] Japanese Unexamined Patent Application, First Publication No. H5-31120
[PTL 2] Japanese Unexamined Patent Application, First Publication No. 2013-138844
[PTL 3] Japanese Unexamined Patent Application, First Publication No. 2012-165812
[PTL 4] PCT International Publication No. WO 2011/043340

### Summary of Invention

### Technical Problem

Being different from gripping forceps which grip biological tissue in a planar manner with a pair of cup-shaped or plate-shaped forcep pieces facing each other, cutting portions of endoscopic scissors are evenly and linearly formed while having narrow widths. In addition, since the biological tissue at a position slightly deviated in the thickness direction of scissor pieces is gripped by using a shearing force, a gripping force thereof is weak. Therefore, in order to precisely incise a desired site with the endoscopic scissors, skill is required. In contrast, as in the endoscopic scissors of PTL 2, when a claw portion is formed at a tip end portion of the scissor piece, an incision can be performed in a state where the claw portion intrudes into biological tissue and grips the biological tissue. Therefore, the scissor pieces can precisely incise target biological tissue without slipping. In addition, as in the endoscopic scissors of PTL 2, when the cutting portions are formed in a serrated manner, a force of gripping biological tissue increases.

However, in the endoscopic scissors of PTL 2, there is a possibility that biological tissue is incised in an excessively deep manner. For example, in a case where mucosal tissue is excised, due to the high invasiveness thereof, there are cases where it is preferable to avoid incising not only the mucosal layer of the surface layer and the submucosal layer which is a lower layer thereof but also the muscle layer which is a further lower layer. In contrast, in the endoscopic scissors of PTL 2, since an incision is performed in a state where the claw portion and a plurality of serrated teeth intrude into the mucosal tissue and grip the mucosal tissue, in a case where the scissor pieces intrude into the mucosal tissue in an excessively deep manner, there is a possibility that the muscle layer is incised as well.

Meanwhile, in the endoscopic scissors disclosed in PTL 3, a recess is formed in the cutting portion and biological tissue locally escapes into the recess. Therefore, the recess functions as a slip-resistant member when an incision is performed. However, since the biological tissue only escapes into the recess within a range of the depth thereof, except the case of incising biological tissue which is flexibly deformed within such a small range, the function of the recess as the slip-resistant member is strictly limited.

The present invention has been made in consideration of the foregoing disadvantages, and there are provided endoscopic scissors, in which scissor pieces can precisely incise target biological tissue without slipping, for example, a minimally invasive technique can be performed without incising the biological tissue in an excessively deep manner reaching the muscle layer.

In addition, in the technology 1 in the related art, as described above, even though the pair of scissor pieces is stably disposed by being supported by the holding frame, there are problems as follows.

That is, when the pair of scissor pieces is opened and closed, there are cases where the outer surfaces of the scissor pieces operating inside the slit are chafed due to contact with the inner surfaces of the holding frame and the insulation coat peels off. Here, due to an opening-closing operation of the pair of scissor pieces, the place where the insulation coat peels off comes to the surface out of the holding frame, leading to a possibility that conductive portions other than the cutting portions come into contact with biological tissue, thereby being a problem.

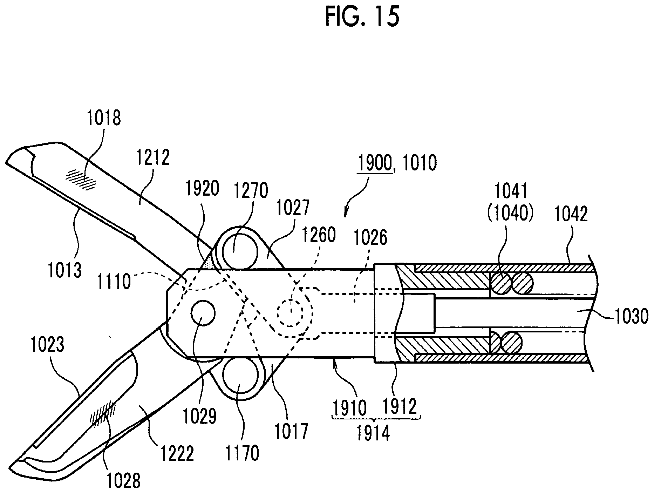

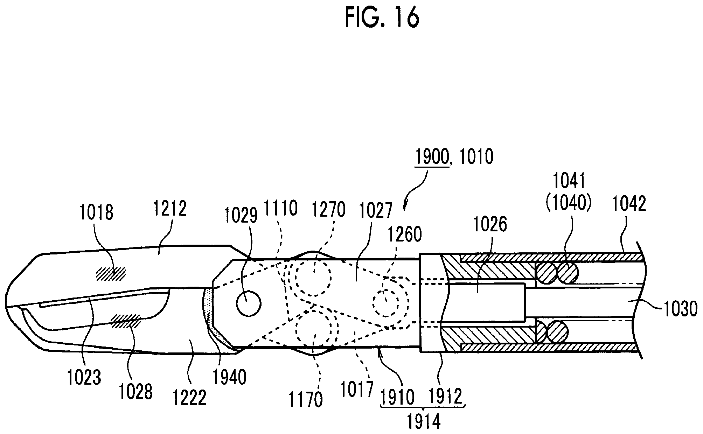

The aforementioned problem will be specifically described by using FIGS. 15 and 16. FIG. 15 is a side view of a tip end portion of a high frequency treatment tool 1900 for an endoscope in the related art and illustrates a pair of scissor pieces 1212 and 1222 in a released state. FIG. 16 is a side view of the tip end portion of the high frequency treatment tool 1900 for an endoscope in the related art and illustrates the pair of scissor pieces 1212 and 1222 in a closed state.

The high frequency treatment tool 1900 for an endoscope illustrated in FIGS. 15 and 16 has a step portions 1110 on a base end side beyond a place which is pivotally supported by a turning pivot 1029 of the scissor pieces 1212 and 1222. The thickness on the base end side beyond the step portions 1110 is formed so as to be smaller than the thickness on a tip end side beyond the step portions 1110. In the high frequency treatment tool 1900 for an endoscope, link pieces 1017 and 1027 and the scissor pieces 1212 and 1222 having thicknesses equal to or less than the height size of the step portions 1110 are mutually and pivotally supported by pivots 1170 and 1270. There is a relationship in which at least the outside surfaces of the scissor pieces 1212 and 1222 and the inner surfaces of arm portions 1910 abut on each other. Therefore, the pair of scissor pieces 1212 and 1222 is stably disposed between the arm portions 1910 facing each other. The arm portion 1910 provided on the depth side in the sheet beyond the scissor piece 1212 is not illustrated. The two arm portions 1910 facing each other configure a holding frame 1914 together with a base end portion 1912.

As illustrated in FIG. 15, when the high frequency treatment tool 1900 for an endoscope is operated from a closed state to a released state, a part of the scissor pieces 1212 and 1222 internally housed in the holding frame 1914 in a closed state comes to the surface as an exposed region 1920 out of the holding frame 1914. In addition, as illustrated in FIG. 16, when the high frequency treatment tool 1900 for an endoscope is operated from a released state to a closed state, a part of the scissor pieces 1212 and 1222 internally housed in the holding frame 1914 in a released state comes to the surface as an exposed region 1940 out of the holding frame 1914. Here, due to operations between a released state and a closed state, there are cases where a portion of the scissor pieces 1212 and 1222 accommodated in the holding frame 1914 comes into contact with the inner surfaces of the holding frame 1914 and is grazed such that insulation coats 1018 and 1028 formed on the outer surfaces peel off. Therefore, the exposed regions 1920 and 1940 in which the insulation coats 1018 and 1028 peel off become conductive regions, leading to a possibility that unscheduled places of biological tissue are cauterized, thereby being a problem.

The above-described problem can also be caused in an endoscopic high frequency treatment tool having gripping pieces other than the pair of scissor pieces (for example, a pair of cup-shaped pieces of which opening portions face each other).

The present invention has been made in consideration of the foregoing disadvantages. That is, according to the present invention, there is provided an endoscopic high frequency treatment tool, in which a treatment portion including a pair of gripping pieces is supported by a holding frame and an insulation coat is prevented from peeling off due to contact between the treatment portion and the holding frame.

### Solution to Problem

According to the present invention, there are provided endoscopic scissors, configured to be inserted into a forceps hole of an endoscope and incise biological tissue. The endoscopic scissors include a pair of scissor pieces that face and overlap each other, are pivotally supported by a turning pivot extending in an overlapping direction so as to be able to be opened and closed, and respectively have cutting portions configured to grip and incise the biological tissue; and an operation wire configured to apply a driving force to base end portion sides of the scissor pieces such that an opening-closing operation of tip end portions of the scissor pieces is performed. At the tip end portion of at least one of the pair of scissor pieces, a claw portion configured to grip the biological tissue is formed to protrude in a closing direction beyond the cutting portion. A concave portion penetrating the cutting portion in a thickness direction is locally formed in the cutting portion of the one or the other scissor piece, and the cutting portion excluding the claw portion and the concave portion is formed so as to have a flat and linear shape extending in a tip end - base end direction.

According to the invention, the claw portion is formed at the tip end portion of the scissor piece so as to protrude in the closing direction, and an incision can be performed in a state where the claw portion intrudes into biological tissue and grips the biological tissue. Therefore, the scissor pieces can precisely incise target tissue without slipping on the biological tissue. In addition, the concave portion formed in the cutting portion penetrates the cutting portion in the thickness direction. Therefore, compared to PTL 3, the cutting portions can intrude into the biological tissue while more biological tissue escapes into the concave portions. Here, excluding the claw portion and the locally formed concave portions, the cutting portions are formed so as to have flat and linear shapes extending in the tip end - base end direction. Therefore, compared to a case where the cutting portions are formed in a serrated manner as in PTL 2, the intrusion of the cutting portions with respect to biological tissue can be suitably restrained. Accordingly, for example, even in a case where the scissor pieces deeply intrude into mucosal tissue, when the scissor pieces are drawn and the mucosal tissue is raised, the muscle layer comes out from between the scissor pieces due to an elastic restoring force, and only the mucosal layer or the submucosal layer is practically incised by the cutting portions. Thus, it is possible to incise the mucosal tissue without damaging the muscle layer.

According to the present invention, there is provided an endoscopic high frequency treatment tool including a conductive operation wire that is inserted through a flexible tube configured to be inserted into a human body, so as to freely advance and retreat; and a treatment portion that includes a pair of gripping pieces which are pivotally supported by a turning pivot so as to be able to mutually perform an opening-closing operation, the gripping pieces including conductive cutting portions and insulation coats coating substantially entire peripheries thereof excluding the cutting portions, and the treatment portion being disposed at a tip end portion of the operation wire; a connection terminal configured to apply a high frequency voltage to the cutting portions; an operation portion that is installed in a base end portion of the flexible tube, and configured to open and close the gripping pieces by operating the treatment portion by performing an advance-retreat operation through the operation wire; a holding frame that partially accommodates the treatment portion on the inner side between a pair of arm portions facing each other and holds both ends of the turning pivot; and a spacer portion that is disposed between an inner surface of the arm portion and a counter-facing surface facing the inner surface of the arm portion in the treatment portion accommodated between the arm portions, and configured to separate the inner surface of the arm portion and the counter-facing surface of the treatment portion from each other.

### Advantageous Effects of Invention

According to the present invention, there is provided the endoscopic scissors, in which the scissor pieces can precisely incise target biological tissue in a minimally invasive manner without slipping.

In addition, according to the endoscopic high frequency treatment tool of the present invention, in a state where the outside surfaces of the treatment portion including the pair of gripping pieces, and the inner surfaces of the holding frame are separated from each other, the treatment portion is supported by the holding frame. Thus, even in a case where the pair of gripping pieces performs an opening-closing operation, the insulation coats formed on the outer surfaces of the treatment portion are prevented from peeling off due to contact with the inner surfaces of the holding frame.

### Brief Description of Drawings

FIG. 1 is a schematic view illustrating the overall structure of endoscopic scissors, according to a first embodiment of the present invention.
FIG. 2 is a longitudinal sectional view of a tip end portion of the endoscopic scissors in a closed state.
FIG. 3 is a longitudinal sectional view of the tip end portion of the endoscopic scissors in a released state.
FIG. 4 illustrates a view describing a state where scissor pieces are released.
FIG. 5 illustrates a view describing a state where the scissor pieces are closed and biological tissue is incised.
FIG. 6 is a side view of the tip end treatment portion in the endoscopic scissors according to a second embodiment of the present invention.
FIG. 7 is a side view of the tip end treatment portion in the endoscopic scissors according to a third embodiment of the present invention.
FIG. 8A is a plan view illustrating a closed state of the tip end treatment portion in the endoscopic scissors, according to a fourth embodiment of the present invention.
FIG. 8B is a side view of the tip end treatment portion in the endoscopic scissors according to the fourth embodiment of the present invention.
FIG. 9 is a side view illustrating a released state of the tip end treatment portion in the endoscopic scissors, according to the fourth embodiment of the present invention.
FIG. 10 is an overall side view of an endoscopic high frequency treatment tool according to a fifth embodiment of the present invention.
FIG. 11 is a side view of a tip end portion of the endoscopic high frequency treatment tool illustrated in FIG. 10 in a closed state.
FIG. 12 is a side view of the tip end portion of the endoscopic high frequency treatment tool illustrated in FIG. 10 in a released state.
FIG. 13A is a partial top view of a treatment portion and a holding frame before being assembled.
FIG. 13B is a partial top view of the treatment portion and the holding frame of the endoscopic high frequency treatment tool.
FIG. 14A is a top view of a treatment portion and a holding frame in an endoscopic high frequency treatment tool according to a sixth embodiment of the present invention before being assembled.
FIG. 14B is a top view partially illustrating the treatment portion and the holding frame of the endoscopic high frequency treatment tool, according to the sixth embodiment.
FIG. 15 is a side view of a tip end portion of an endoscopic high frequency treatment tool in the related art and illustrates a pair of scissor pieces in a released state.
FIG. 16 is a side view of the tip end portion of the endoscopic high frequency treatment tool in the related art and illustrates the pair of scissor pieces in a closed state.
FIG. 17A is a plan view illustrating a closed state of a tip end treatment portion in endoscopic scissors, according to a seventh embodiment of the present invention.
FIG. 17B is a side view of the tip end treatment portion in the endoscopic scissors according to the seventh embodiment of the present invention.
FIG. 18 is a side view illustrating a released state of the tip end treatment portion in the endoscopic scissors, according to the seventh embodiment of the present invention.

### Description of Embodiments

Hereinafter, embodiments of the present invention will be described based on the drawings. In each of the drawings, common reference signs will be applied to the corresponding configuration elements, and overlapping description will be appropriately omitted.

### <First Embodiment>

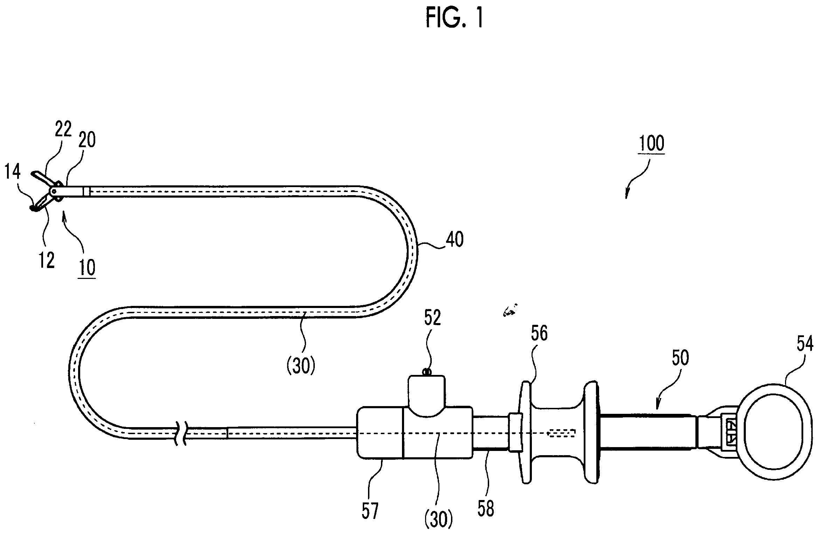

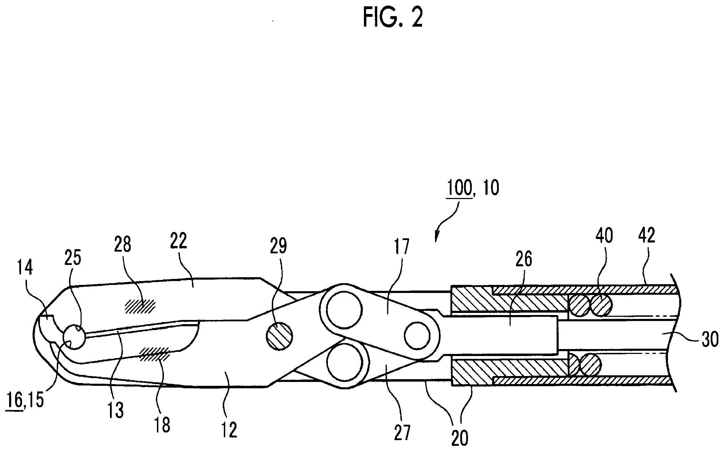

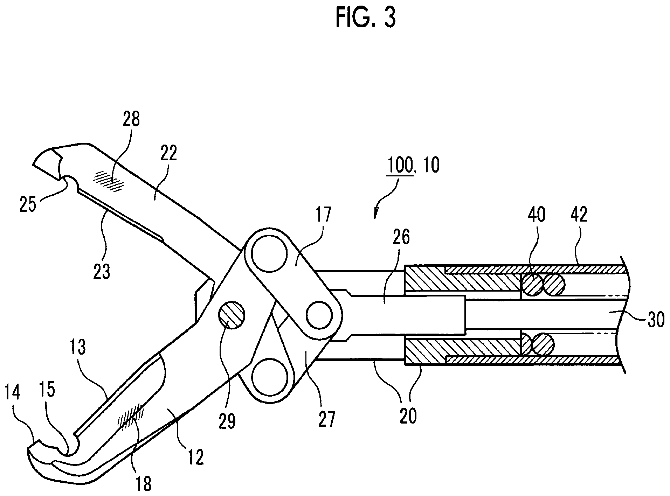

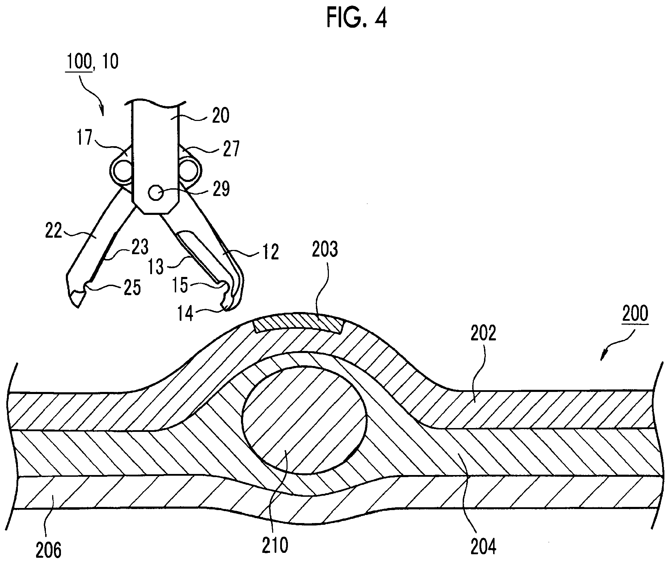

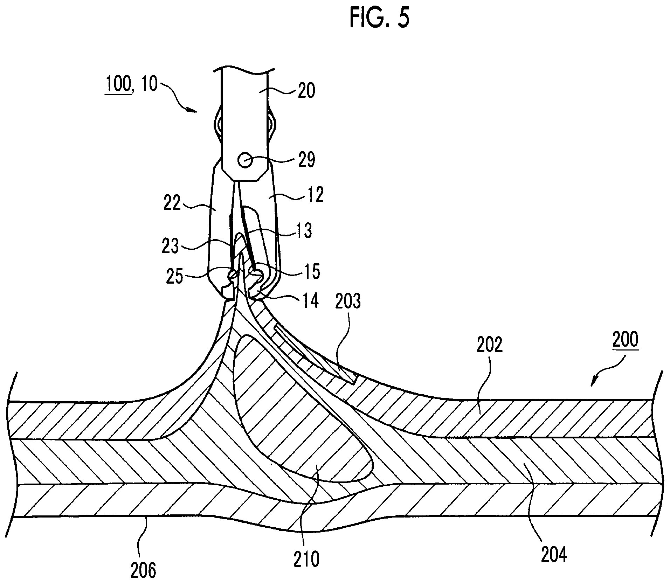

FIG. 1 is a schematic view illustrating the overall structure of scissors 100 for an endoscope, according to a first embodiment of the present invention. FIG. 2 is a longitudinal sectional view of a tip end portion of the scissors 100 for an endoscope in a closed state, and FIG. 3 is a longitudinal sectional view of the tip end portion of the scissors 100 for an endoscope in a released state. FIG. 4 illustrates a view describing a state where scissor pieces 12 and 22 are released. FIG. 5 illustrates a view describing a state where the scissor pieces 12 and 22 are closed and biological tissue 200 is incised.

First, the outline of the scissors 100 for an endoscope of the present embodiment will be described. The scissors 100 for an endoscope are instruments which are used by being inserted into a forceps hole (not illustrated) of an endoscope and incise the biological tissue 200 (refer to FIGS 4 and 5). The scissors 100 for an endoscope include a pair of the scissor pieces 12 and 22 that face and overlap each other and are pivotally supported by a turning pivot 29 (refer to FIGS 2 and 3) extending in an overlapping direction so as to be able to be opened and closed, and an operation wire 30. The pair of scissor pieces 12 and 22 individually has cutting portions 13 and 23 gripping and incising the biological tissue 200. The operation wire 30 is a member which allows a driving force to be applied to base end portion sides of the scissor pieces 12 and 22 such that an opening-closing operation of the tip end portions of the scissor pieces 12 and 22 is performed. At the tip end portion of at least one of the pair of scissor pieces 12 and 22, a claw portion 14 gripping the biological tissue 200 is formed so as to protrude in a closing direction beyond the cutting portion 13. In the scissors 100 for an endoscope of the present embodiment, concave portions 15 and 25 respectively penetrating the cutting portions 13 and 23 in a thickness direction are locally formed in the cutting portions 13 and 23 of the one or the other scissor piece (in the present embodiment, both the scissor pieces 12 and 22), and the cutting portions 13 and 23 excluding the claw portion 14 and the concave portions 15 and 25 are formed so as to have flat and linear shapes extending in a tip end - base end direction.

Subsequently, the scissors 100 for an endoscope of the present embodiment will be described in detail.

The scissors 100 for an endoscope have a scissor-shaped tip end treatment portion 10 gripping the biological tissue 200 by opening and closing the thin plate-shaped scissor pieces 12 and 22 overlapping each other and applying a shearing force to the biological tissue 200. The scissor pieces 12 and 22 are mutually opened and closed in an in-plane direction while having the turning pivot 29 as a pivot. An extending direction (pivot direction) of the turning pivot 29 is the overlapping direction of the scissor pieces 12 and 22, in other words, the thickness direction of the scissor pieces 12 and 22. The tip end treatment portion 10 is driven through the operation wire 30 so as to be opened and closed. The operation wire 30 is made of a conductive metal material such as stainless steel.

The scissors 100 for an endoscope include a flexible sheath 40 and a hand operation portion 50 which is provided on a proximal side of the sheath 40. The sheath 40 has an insulation tube shape, and the operation wire 30 is inserted through the inside thereof.

The hand operation portion 50 includes a shaft portion 58 through which the operation wire 30 is inserted, a finger hook ring 54 which is provided in a base end portion of the shaft portion 58, and a slider 56 with which a base end of the operation wire 30 is interlocked and which advances and retreats with respect to the shaft portion 58. The operation wire 30 is inserted through so as to be slidable with respect to the shaft portion 58. For example, a user inserts a thumb into the finger hook ring 54, and the slider 56 is driven with two other fingers so as to advance and retreat along a longitudinal direction of the shaft portion 58. Accordingly, the operation wire 30 advances or retreats with respect to the hand operation portion 50. The operation wire 30 is inserted through so as to be able to advance and retreat with respect to the sheath 40. In association with a movement of the slider 56, the tip end portion of the operation wire 30 advances or retreats with respect to the sheath 40.

As illustrated in FIGS. 2 and 3, on a tip end side of the operation wire 30, an advance/retreat portion 26 is integrally interlocked. Two link pieces 17 and 27 are turnably interlocked with the advance/retreat portion 26. Moreover, a first scissor piece 12 is turnably interlocked with the link piece 17, and in the link piece 27, a second scissor piece 22 is turnably interlocked. The first scissor piece 12, the second scissor piece 22, and the link pieces 17 and 27 relatively turn within a plane illustrated in FIGS. 2 and 3.

As illustrated in FIGS. 1 to 3, in the tip end of the sheath 40, a holding frame 20 is mounted. The base end portion of the holding frame 20 is inserted into the sheath 40 and is fixed, and the tip end portion of the holding frame 20 protrudes beyond the sheath 40 and accommodates the link pieces 17 and 27. The turning pivot 29 which causes the first scissor piece 12 and the second scissor piece 22 to be turnably interlocked with each other is fixed to the holding frame 20.

As illustrated in FIG. 2, when the operation wire 30 and the advance/retreat portion 26 are drawn to the base end side (to the right, in FIG. 2), the link pieces 17 and 27 and the scissor pieces 12 and 22 have substantially linear shapes, and the tip end treatment portion 10 is in a closed state. On the contrary, as illustrated in FIG. 3, when the operation wire 30 and the advance/retreat portion 26 are pushed out to the tip end side (to the left, in FIG. 3), the link pieces 17 and 27 approach the turning pivot 29 inside the holding frame 20 so as to drive the scissor pieces 12 and 22, and the tip end treatment portion 10 is in a released state.

In the vicinity of the turning pivot 29, the scissor pieces 12 and 22 have substantial L-shapes obtusely curved in an in-turning plane direction, that is, sickle shapes. The shapes of scissor pieces 12 and 22 forming a pair may be symmetric or asymmetric. The scissor pieces 12 and 22 and the link pieces 17 and 27 configure a four-bar link mechanism by which the tip end treatment portion 10 is driven so as to be opened and closed while having the turning pivot 29 as a fulcrum. The operation wire 30 allows a driving force to be applied to the base end portions of the scissor pieces 12 and 22 via the advance/retreat portion 26 and the link pieces 17 and 27, and an opening-closing operation of the tip end portions of the scissor pieces 12 and 22 is performed.

The sheath 40 of the present embodiment is a coil made by tightly winding a conductive wire such as a stainless steel wire. On the outside surface of the sheath 40, an insulation coat 42 is tightly provided. The operation wire 30 is connected to a rotation operation portion 57 (refer to FIG. 1) while having friction therewith. When the rotation operation portion 57 is subjected to a shaft-rotation around the shaft portion 58, the operation wire 30 of which the base end is fixed to the slider 56 rotates inside the sheath 40. Accordingly, the tip end treatment portion 10 can be directed in a desired orientation. The rotation operation portion 57 is rotatably attached to a power supply portion 52. In a state where a power supply cable (not illustrated) causing the power supply portion 52 and a high frequency power source (not illustrated) to be interlocked with each other is suspended downward, the rotation operation portion 57 can be operated so as to rotate around the shaft portion 58. In place of the present embodiment, the slider 56 may be configured to be able to be subjected to a shaft-rotation around the shaft portion 58, and the function of the rotation operation portion 57 may be applied to the slider 56. That is, the slider 56 may be configured to be driven so as to advance and retreat along the longitudinal direction of the shaft portion 58 such that the operation wire 30 advances and retreats and an opening-closing operation of the tip end treatment portion 10 is performed. In addition, the slider 56 may be configured to be subjected to a shaft-rotation around the shaft portion 58 such that the tip end treatment portion 10 rotates so as to be directed in a desired orientation.

In the scissor pieces 12 and 22, the cutting portions 13 and 23 are formed along end edges (edges) facing each other in each of the inner surfaces overlapping each other. The cutting portions 13 and 23 of the present embodiment are formed in a part of a region on the tip end side beyond the turning pivot 29 in the scissor pieces 12 and 22. The cutting portions 13 and 23 are cutting faces which are formed so as to be comparatively sharp in the scissor pieces 12 and 22 and are sites incising the biological tissue 200 (refer to FIGS 4 and 5). Specifically, when the slider 56 (refer to FIG. 1) of the hand operation portion 50 is drawn and the scissor pieces 12 and 22 are turned while having the turning pivot 29 as the center such that the tip end portion is closed as illustrated in FIG. 2, a shearing force is generated between the cutting portions 13 and 23.

The end edges of the cutting portions 13 and 23 may be sharply formed to the extent that the biological tissue 200 can be incised by such a shearing force, or the end edges thereof may be formed to have obtuse angles to the extent that the biological tissue 200 is not incised by such a shearing force. In the scissors 100 for an endoscope of the present embodiment, the end edges of the cutting portions 13 and 23 are formed to have obtuse angles and have a configuration in which the biological tissue 200 is not practically incised by only the shearing force which is generated when the slider 56 is drawn. In a state where the scissor pieces 12 and 22 grip the biological tissue 200, when a high frequency voltage (will be described later) is applied to both the scissor pieces 12 and 22, the biological tissue 200 is cauterized and incised. Accordingly, while the biological tissue 200 is gripped by the scissor pieces 12 and 22 and the biological tissue 200 is stretched so as to be elongated with a predetermined tensile force, the biological tissue 200 is not unexpectedly incised.

In at least one tip end portion of the scissor piece 12 or 22, the claw portion 14 for gripping the biological tissue 200 is formed so as to protrude in the closing direction beyond the cutting portion 13 or 23. The closing direction indicates a direction oriented from the scissor pieces 12 or 22 on one side to the scissor pieces 22 or 12 on the other side, and the opposite direction will be called an opening direction.

As illustrated in FIG. 3, in the scissors 100 for an endoscope of the present embodiment, the claw portion 14 is formed in the tip end portion of the first scissor piece 12 so as to protrude in the closing direction, and no such claw portion is formed in the second scissor piece 22. However, in place of the present embodiment, in both the first scissor piece 12 and the second scissor piece 22, claw portions (reference signs omitted) respectively protruding inward may be formed. In a case where claw portions are respectively formed in the first scissor piece 12 and the second scissor piece 22, the position, the shape, and the size of each claw portion are not particularly limited. Claw portions having the same shapes or shapes different from each other may protrude inward from the scissor pieces 12 and 22 respectively.

As illustrated in FIG. 5, the claw portion 14 is a projection portion which intrudes into the biological tissue 200 and holds the biological tissue 200 gripped by the scissor pieces 12 and 22 so as to wrap the biological tissue 200, thereby preventing the biological tissue 200 from being dropped. No cutting face is formed in the claw portion 14, and the thickness size thereof is greater than the thickness size of the cutting portion 13.

In the cutting portion 13 of the first scissor piece 12, the concave portion 15 penetrating the cutting portion 13 in the thickness direction is locally formed. The cutting portion 13 excluding the claw portion 14 and the concave portion 15 is formed so as to have a flat and linear shape extending in the tip end - base end direction (lateral direction in FIG. 2). In addition, in the cutting portion 23 of the second scissor piece 22 as well, the concave portion 25 penetrating the cutting portion 23 in the thickness direction is locally formed. The cutting portion 23 excluding the concave portion 25 is formed so as to have a flat and linear shape extending in the tip end - base end direction (lateral direction in FIG. 2).

The state where the cutting portions 13 and 23 have flat and linear shapes indicates that the end edges which apply a shearing force to the biological tissue 200 in each of the inner surfaces of the scissor pieces 12 and 22 overlapping each other have straight line shapes or smoothly bent shapes. That is, in the cutting portions 13 and 23 of the present embodiment, no such keen protrusion portion like crocodile teeth is formed. The cutting portion 13 has a straight line shape excluding the forming region of the claw portion 14 and the concave portion 15. Similarly, the cutting portion 23 has a straight line shape excluding the forming region of the concave portion 25. However, in place of the present embodiment, one or both the cutting portions 13 and 23 may be smoothly bent so as to have convex arc shapes protruding in the closing direction or concave arc shapes recessed in the opening direction.

In at least one of the cutting portions 13 and 23 in the scissor piece 12 or 22 (both, in the present embodiment), the concave portions 15 and 25 penetrating the cutting portions 13 and 23 in the thickness direction are locally formed. The state where the concave portions 15 and 25 are formed in the cutting portions 13 and 23 indicates that the concave portions 15 and 25 are formed so as to be recessed in the opening direction from the end edges incising the biological tissue 200. In other words, the concave portions 15 and 25 are open in the end edges incising the biological tissue 200. In the scissors 100 for an endoscope of the present embodiment, an aspect in which the concave portions 15 and 25 are respectively formed in both the cutting portions 13 and 23 is exemplified. However, as in a second embodiment (will be described later), the concave portion 15 may be formed in only one of the cutting portions 13 and 23 (for example, the cutting portion 13).

The concave portions 15 and 25 respectively penetrate the cutting portions 13 and 23 in the thickness direction. Therefore, being different from a case where a bottomed recess is formed in the cutting portion as in the endoscopic scissors of PTL 3, as illustrated in FIG. 5, the biological tissue 200 can escape into the concave portions 15 and 25 and the scissor pieces 12 and 22 can more favorably intrude into the biological tissue 200.

The concave portions 15 and 25 of the present embodiment respectively have substantially semicircular shapes. The substantially semicircular shape indicates a concave shape configured with a smoothly bent line.

The concave portion 15 is formed by being concatenated with the claw portion 14 which is formed in the tip end portion of the scissor piece 12. That is, the claw portion 14 is protrudingly formed so as to rise from an inner peripheral surface of the concave portion 15 in the closing direction. The cutting portion 13 of the scissor piece 12 is formed so as to extend to the base end side beyond the concave portion 15 and to have a linear shape.

Such substantially semicircular-shaped concave portions 15 and 25 are respectively formed at mutually corresponding positions in the cutting portions 13 and 23 of the pair of scissor pieces 12 and 22 so as to face each other. When the scissor pieces 12 and 22 are closed, the concave portions 15 and 25 meet together and a substantially circular-shaped penetration hole 16 is formed (refer to FIG. 2).

When the scissor pieces 12 and 22 are closed, the penetration hole 16 is formed. Accordingly, the biological tissue 200 can be gripped by the scissor pieces 12 and 22 so as to be wrapped by the penetration hole 16 in a columnar manner. Therefore, in cooperation with the claw portion 14 which has intruded into the biological tissue 200, the biological tissue 200 can be gripped at a plurality of places such as the claw portion 14 and the penetration hole 16. Accordingly, even in a case where a force is applied from the biological tissue 200 to the tip end treatment portion 10 not only in the tip end - base end direction but also in the intersecting direction thereof, the tip end treatment portion 10 is preferably prevented from dropping the grabbed biological tissue 200.

As illustrated in FIG. 4, examples of the biological tissue 200 include mucosal tissue in which a submucosal layer 204 is stacked on the top surface of a muscle layer 206, and a mucosal layer 202 is additionally stacked on the top surface thereof. Examples of such biological tissue 200 include an inner wall of a body cavity in a digestive system such as the inner wall surface of the stomach. In the mucosal layer 202, lesion tissue 203 is locally formed. In the submucosal layer 204, in a region corresponding to a lower portion of the lesion tissue 203, a swelling site 210 having a physiological saline solution, hyaluronic acid, or the like injected is formed in advance. When the swelling site 210 is formed, the lesion tissue 203 is greatly isolated from the muscle layer 206. The scissors 100 for an endoscope of the present embodiment incise the lesion tissue 203 so as to surround the peripheral edge thereof and excise the mucosal layer 202 and the submucosal layer 204 including the lesion tissue 203 from the biological tissue 200.

Since the concave portions 15 and 25 are locally formed with respect to the cutting portions 13 and 23, in a state where the claw portion 14 and the concave portions 15 and 25 (penetration hole 16) intrude into the mucosal layer 202 of the biological tissue 200, the flat and linear cutting portions 13 and 23 come into contact with the mucosal layer 202. Therefore, the scissor pieces 12 and 22 are restrained from excessively intruding into the mucosal layer 202. In other words, since the cutting portions 13 and 23 evenly press the mucosal layer 202, the claw portion 14 and the penetration hole 16 are suitably restrained from intruding into the mucosal layer 202. When the tip end treatment portion 10 gripping the biological tissue 200 is raised upward, in a state where the mucosal layer 202 is caught by the claw portion 14, the biological tissue 200 can slightly slide off in the tip end - base end direction along the evenly formed cutting portions 13 and 23. Accordingly, the mucosal layer 202 and the submucosal layer 204 are in a state of being gripped by the tip end treatment portion 10, are stretched, and are elongated with a predetermined tensile force. Meanwhile, the muscle layer 206 is separated from the tip end treatment portion 10. In such a state, the slider 56 (refer to FIG. 1) is further drawn such that the scissor pieces 12 and 22 are closed. As described below, by applying a high frequency voltage, the mucosal layer 202 (or the mucosal layer 202 and the submucosal layer 204) is incised. Since a tensile force is applied to the mucosal layer 202 and the submucosal layer 204, a shearing force of the cutting portions 13 and 23 is straightly transmitted to the mucosal layer 202 in a depth direction, and thus, the mucosal layer 202 can be precisely incised at a desired position.

The biological tissue 200 may be incised in the depth direction with the scissors 100 for an endoscope through a plurality of times. That is, after the mucosal layer 202 is incised, the tip end treatment portion 10 may be more deeply inserted into the biological tissue 200 so as to incise the submucosal layer 204. Moreover, the submucosal layer 204 below the swelling site 210 may be incised in the horizontal direction along the boundary surface between the submucosal layer 204 and the muscle layer 206.

In the present embodiment, an aspect in which the concave portions 15 and 25 are formed so as to face each other and a substantially circular-shaped penetration hole 16 is formed by closing the scissor pieces 12 and 22 is exemplified. However, the concave portions 15 and 25 are not limited thereto. The concave portions 15 and 25 may be respectively formed in the scissor pieces 12 and 22 at positions different from each other in the tip end - base end direction. In this case, the concave portions 15 and 25 may be formed at positions such that parts thereof are arranged in a row, or the concave portions 15 and 25 may be formed at positions completely separated from each other.

The tip end treatment portion 10 of the present embodiment performs an incision by applying a shearing force while cauterizing the biological tissue 200. Accordingly, the biological tissue 200 can be incised with a small force. In addition, bleeding of the incised site can be stopped at the same time.

As illustrated in FIG. 1, the scissors 100 for an endoscope includes the power supply portion 52 which applies a high frequency voltage to the scissor pieces 12 and 22 through the operation wire 30. The power supply portion 52 is a terminal connected to the high frequency power source (not illustrated).

All of the scissor pieces 12 and 22, the link pieces 17 and 27, and the advance/retreat portion 26 configuring the tip end treatment portion 10 are made of conductive metal materials. In addition, as described above, the operation wire 30 is also made of a conductive metal material. Therefore, a high frequency voltage applied to the power supply portion 52 is applied to the scissor pieces 12 and 22.

As illustrated in FIG. 3, the outer surfaces of the pair of scissor pieces 12 and 22 are respectively provided with insulation coats 18 and 28. For convenience, in FIG. 3, the insulation coats 18 and 28 are partially illustrated. However, the insulation coats 18 and 28 are formed in the scissor pieces 12 and 22 substantially in their entirety, excluding the cutting portions 13 and 23 and the concave portions 15 and 25. That is, the inner peripheral surfaces of the cutting portions 13 and 23 and the concave portions 15 and 25 are exposed from the insulation coats 18 and 28.

The insulation coats 18 and 28 can be formed by performing coating with insulation materials, for example, ceramic materials such as a fluororesin, diamond-like carbon (DLC), a titanium oxide-based material, and a silicon-based material. According to an examination of the inventor, it has been found that particularly when a silicon-based ceramic material is selected as the insulation material, it is possible to acquire the insulation coats which are particularly excellent in insulation, durability with respect to electrification, and adhesion. The insulation and the durability are achieved by increasing the coating thicknesses of the insulation coats. However, in a case where the coating thickness is increased, there is a disadvantage in that adhesion with respect to the scissor pieces is deteriorated. In contrast, when silicon-based ceramic is selected as the insulation material, compared to other ceramic materials, the fluororesin, and the DLC, insulation, durability with respect to electrification, and adhesion can be improved in a well-balanced state.

In a case where the silicon-based ceramic is adopted as the insulation material, the insulation coats 18 and 28 can be formed by paining the scissor pieces 12 and 22 with liquid insulation coating including polysiloxane (organo-polysiloxane). As the insulation coating, in addition to polysiloxane, an inorganic filler such as silica, and a coloring agent such as a binder resin and a pigment may be mixed with an aqueous menstruum or a solvent-based menstruum, or a hardening agent may be arbitrarily mixed.

The insulation coats 18 and 28 respectively provided on the outer surfaces of the pair of scissor pieces 12 and 22 may be colored with colors different from each other. Specifically, it is favorable that a first pigment which is mixed into the insulation coat 18 painted on the scissor piece 12 and a second pigment which is mixed into the insulation coat 28 painted on the scissor piece 22 are made of different-type materials exhibiting colors different from each other to the extent of being able to be visually discriminated. Accordingly, under an endoscopic observation, the orientations of the front and the back of the scissor pieces 12 and 22 can be easily grasped.

The thicknesses of the insulation coats 18 and 28 are not particularly limited. However, in a case where a fluororesin is adopted as the insulation material, it is preferable that the content ranges from 20 µm to 80 µm. In a case of adopting DLC, it is preferable that the content ranges from 1 µm to 5 µm. In a case of adopting a silicon-based ceramic material, it is preferable that the content ranges from 5 µm to 40 µm.

The cutting portions 13 and 23 exposed from the insulation coats 18 and 28 serve as linear-shaped electrodes. The scissor pieces 12 and 22 serve as monopolar-type high-frequency electrodes when a high frequency voltage having the coordinate phase is applied from the power supply portion 52.

When the cutting portions 13 and 23 are conductive electrodes, and the insulation coats 18 and 28 are formed in different regions in the scissor pieces 12 and 22, the insulation coats 18 and 28 can be formed as follows. That is, the scissor pieces 12 and 22 are coated with an insulation material in advance in their entirety. Thereafter, the coating in curve-lined regions corresponding to the inner peripheral surfaces of the cutting portions 13 and 23 and the concave portions 15 and 25 can be caused to peel off by performing polishing or using chemicals.

In the present embodiment, an aspect in which the substantially semicircular shaped inner peripheral surfaces of the concave portions 15 and 25 are generally exposed from the insulation coats 18 and 28 and are used as parts of the electrodes is exemplified. However, the present invention is not limited thereto. The insulation coats 18 and 28 may be formed on the inner peripheral surfaces of the concave portions 15 and 25.

As in the present embodiment, when the insulation coats 18 and 28 is exposed instead of being formed on the inner peripheral surfaces of the concave portions 15 and 25, the biological tissue 200 can be cauterized by the cutting portions 13 and 23 and the concave portions 15 and 25. Therefore, it is possible to promptly perform an incision technique. On the contrary, when the insulation coats 18 and 28 are formed on the inner peripheral surfaces of the concave portions 15 and 25, and the biological tissue 200 is cauterized, coagulated tissue is prevented from adhering to the insides of the concave portions 15 and 25. Therefore, in a case where the biological tissue 200 is incised over a plurality of places by using the scissors 100 for an endoscope, the concave portions 15 and 25 are not filled with coagulated tissue. Thus, a strong gripping force of the scissor pieces 12 and 22 is prevented from being deteriorated.

The holding frame 20 is made of a metal material at least of which the outside surface is coated with an insulation coat, or an insulation material such as a ceramic material and a resin material. That is, a high frequency voltage applied to the power supply portion 52 is not unexpectedly applied to the biological tissue 200 (refer to FIGS 4 and 5) through the holding frame 20. Accordingly, the cauterized biological tissue 200 is prevented from adhering to not only the scissor pieces 12 and 22 excluding the cutting portions 13 and 23 but also the holding frame 20.

### <Second Embodiment>

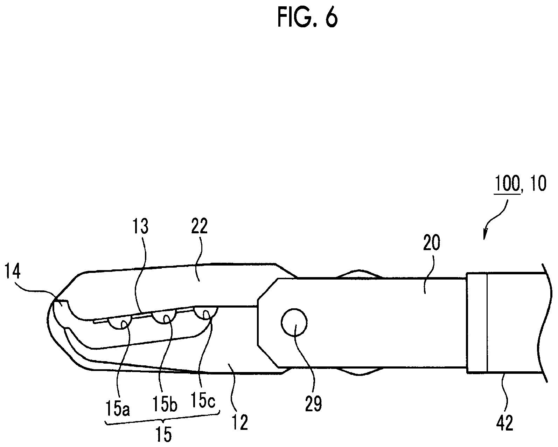

FIG. 6 is a side view of the tip end treatment portion 10 in the scissors 100 for an endoscope according to the second embodiment of the present invention. FIG. 6 illustrates the tip end treatment portion 10 in a closed state.

In the scissors 100 for an endoscope of the present embodiment, a plurality of concave portions 15 (15a to 15c) are formed in at least any cutting portion 13 of one or the other of the pair of scissor pieces 12 and 22 (for example, the scissor piece 12) so as to be arranged while being separated from each other in the tip end - base end direction. The present embodiment is different from the first embodiment in that the point that a remaining length region excluding the forming region of the concave portion 15a to 15c is formed so as to have a continuously linear shape in the scissor piece 12.

Hereinafter, the description overlapping the first embodiment will be appropriately omitted.

As in the scissors 100 for an endoscope of the second embodiment, it is possible to acquire a strong gripping force with respect to the biological tissue 200 (refer to FIG. 5) by forming the plurality of concave portions 15a to 15c in the scissor piece 12. In the scissor piece 12, the claw portion 14 is formed so as to protrude in the closing direction beyond the cutting portion 13. In the cutting portion 13 or 23, on a side where the claw portion 14 is formed (cutting portion 13), the plurality of concave portions 15a to 15c are formed. When the claw portion 14 and the concave portion 15a to 15c are formed, the gripped the biological tissue 200 can be favorably restrained from dropping from the tip end treatment portion 10.

The concave portion 15a to 15c respectively have substantially semicircular shapes. The radii of curvature and the depths of the concave portion 15a to 15c are not particularly limited. However, in the present embodiment, the concave portion 15a to 15c share the common radius of curvature and the common depth.

In this case, the concave portion 15a to 15c are formed so as to be separated from each other, and each of the flat and linear cutting portions 13 is formed between spaces in the concave portion 15a to 15c. Accordingly, the scissor piece 12 is restrained from excessively intruding into the biological tissue 200. In addition, when no concave portion 25 (refer to FIG. 2) is formed in the scissor piece 22 of the other, and counter-facing positions of the concave portion 15a to 15c are evenly formed, the scissor piece 12 is similarly restrained from excessively intruding into the biological tissue 200. Accordingly, the muscle layer 206 (refer to FIG. 5) in the biological tissue 200 which is strongly engaged with the scissor piece 12 mainly slides off from the side of the scissor piece 22. Therefore, without incising the muscle layer 206, the mucosal layer 202 can be favorably gripped by the scissor piece 12, and the mucosal layer 202 can be cauterized and incised.

In addition to between the concave portion 15a and the concave portion 15b and between the concave portion 15b and the concave portion 15c, the conductive cutting portion 13 exposed from the insulation coat 18 (refer to FIG. 3) is formed between the claw portion 14 and the concave portion 15a as well. Accordingly, in a state where the claw portion 14 and the concave portion 15a to 15c intrude into the biological tissue 200, the biological tissue 200 is cauterized and incised at sites between thereof.

In place of the second embodiment, the depths of the concave portion 15a to 15c may be different from each other. Specifically, the concave portion 15a positioned on the tip end side of the scissor piece 12 may be formed so as to be deeper than the concave portion 15b in the middle, and the concave portion 15c positioned on the base end side of the scissor piece 12 may be formed so as to be shallower than the concave portion 15b. Since the scissor pieces 12 and 22 pivotally turn while having the turning pivot 29 as the center, and the tip end sides thereof are opened wider than the base end sides, much biological tissue 200 is gripped by the scissor pieces 12 and 22 on the tip end side (refer to FIG. 5). Therefore, when the concave portion 15a on the tip end side is caused to be deep and the concave portion 15c on the base end side is caused to be shallow, the concave portion 15a to 15c can acquire equal engagement force with respect to the biological tissue 200.

### <Third Embodiment>

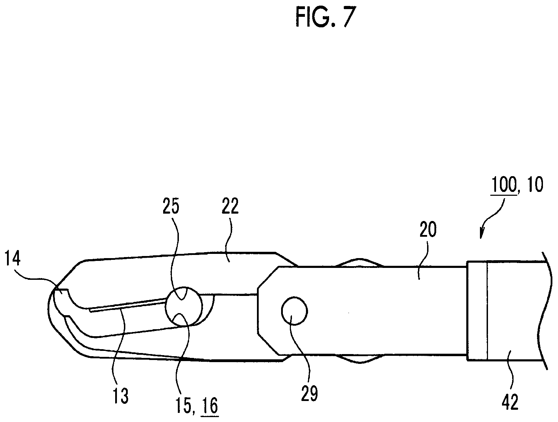

FIG. 7 is a side view of the tip end treatment portion 10 in the scissors 100 for an endoscope according to a third embodiment of the present invention. FIG. 7 illustrates the tip end treatment portion 10 in a closed state.

The scissors 100 for an endoscope of the present embodiment is different from those of the first embodiment in that the concave portions 15 and 25 are formed on the base end side beyond an intermediate portion of the scissor pieces 12 and 22 in the tip end - base end direction.

The intermediate portion of the scissor pieces 12 and 22 mentioned herein indicates the middle of the length in the tip end - base end direction from each of the tip ends of the scissor pieces 12 and 22 to the turning pivot 29, and a region driven by the link pieces 17 and 27 on the base end side beyond the turning pivot 29 is not considered. In addition, the state where the concave portions 15 and 25 are formed on the base end side beyond the intermediate portion of the scissor pieces 12 and 22 in the tip end - base end direction indicates that the middle position of the concave portions 15 and 25 is on the base end side beyond the intermediate portion of the scissor pieces 12 and 22 in the tip end - base end direction.

Hereinafter, the description overlapping the first embodiment or the second embodiment will be appropriately omitted.

In the scissor piece 12, the claw portion 14 is formed so as to protrude in the closing direction beyond the cutting portion 13. Between the claw portion 14 and the concave portion 15, the flat and linear-shaped cutting portion 13 is formed so as to be exposed from the insulation coat 18 (refer to FIG. 3). Similarly, the scissor piece 22 (refer to FIG. 3) of the cutting portion 23 is also formed on the tip end side of the concave portion 25.

The concave portions 15 and 25 have substantially semicircular shapes and are respectively formed at mutually corresponding positions in the cutting portions 13 and 23 of the pair of scissor pieces 12 and 22 so as to face each other. The present embodiment shares a common point with the first embodiment in that when the scissor pieces 12 and 22 are closed, the concave portions 15 and 25 meet together and a substantially circular-shaped penetration hole 16 is formed.

The forming depth of the concave portion 15 formed in the scissor piece 12 is equal to or greater than the protrusion height of the claw portion 14 protruding in the closing direction from the cutting portion 13. Accordingly, much biological tissue 200 (refer to FIG. 5) escapes into the penetration hole 16 and the scissor pieces 12 and 22 can intrude into the biological tissue 200. Therefore, when the tip end treatment portion 10 is raised in a state where the biological tissue 200 is gripped, in the claw portion 14 at the tip end of the scissor piece 12, and between the concave portions 15 and 25 formed on the base end side beyond the intermediate portion, a tensile force can be applied to the biological tissue 200 (mucosal layer 202). That is, when the concave portions 15 and 25 are formed on the base end side beyond the intermediate portion and the cutting portions 13 and 23 are formed exclusively on the tip end side beyond the concave portions 15 and 25, a tensile force can be applied to the length region substantially in its entirety incised by the scissor pieces 12 and 22 within the biological tissue 200.

### <Fourth Embodiment>

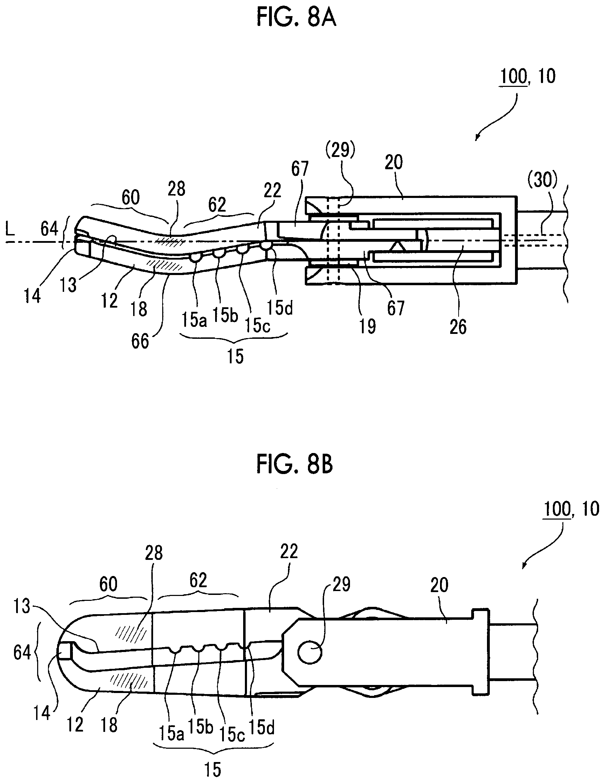

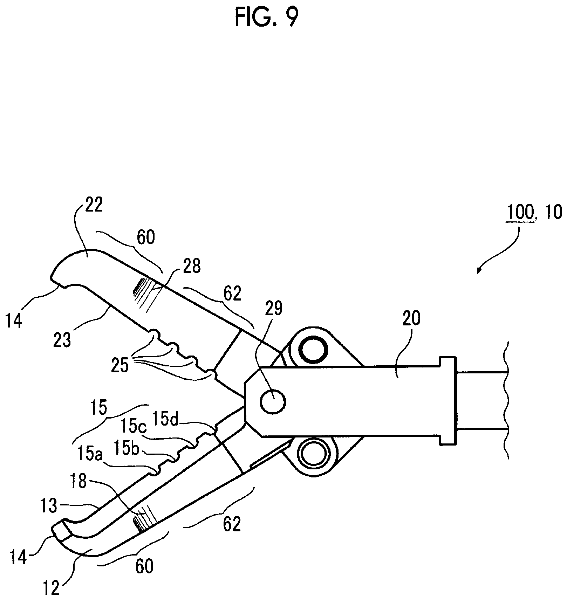

FIG. 8A is a plan view illustrating a closed state of the tip end treatment portion 10 in the scissors 100 for an endoscope, according to a fourth embodiment. FIG. 8B is a side view of the tip end treatment portion 10. FIG. 9 is a side view illustrating a released state of the tip end treatment portion 10 in the scissors 100 for an endoscope, according to a fourth embodiment. The description overlapping any of the first to third embodiments described above will be appropriately omitted.

The present embodiment is different from the first embodiment in that the pair of scissor pieces 12 and 22 in the scissors 100 for an endoscope of the present embodiment individually has a tip end bent portion 60 which is bent toward one side (upward, in FIG. 8A) in the overlapping direction, on the tip end side beyond the forming region of the concave portions 15a to 15d.

The tip end bent portion 60 can be made by bend-forming the plate-shaped scissor pieces 12 and 22 in a plane-perpendicular direction. More specifically, when the intermediate portion of the scissor pieces 12 and 22 is subjected to bend-forming in the plane-perpendicular direction and a bent projection portion 66 is formed, tip ends 64 of the scissor pieces 12 and 22 are oriented in an oblique direction with respect to an extension line L of the operation wire 30. The extension line L is a virtual line in the scissors 100 for an endoscope and is illustrated with a two-dot chain line in FIG. 8A.

The bent projection portion 66 is a convex region formed on a side (lower side, in FIG. 8A) opposite to the bending direction of the tip end bent portions 60 in the scissor pieces 12 and 22. The radii of curvature of the scissor pieces 12 and 22 in the bent projection portion 66 are not particularly limited. However, it is preferable that the tip end bent portions 60 and base end bent portions 62 are formed so as to be smoothly continuous at the bent projection portion 66. As in the present embodiment, since the tip end bent portions 60 are formed, when the tip end treatment portion 10 advances along the mucosal layer 202 (refer to FIG. 4), the bent projection portion 66 abuts on the mucosal layer 202. Therefore, the tip end 64 of the tip end treatment portion 10 is oriented to a side opposite to the mucosal layer 202. Thus, the tip end 64 is restrained from interfering with the mucosal layer 202.

In the scissors 100 for an endoscope of the present embodiment, the pair of scissor pieces 12 and 22 individually has the base end bent portion 62 which is bent toward the other side (downward, in FIG. 8A) in the overlapping direction, on the base end side beyond the tip end bent portion 60. The tip ends 64 of the pair of scissor pieces 12 and 22 are positioned on the extension line L of the operation wire 30. In addition, when the scissors 100 for an endoscope are viewed from the tip end side, the scissor pieces 12 and 22 including the tip end bent portions 60 and the base end bent portions 62 are stored inside an envelope region of the holding frame 20. Accordingly, when the scissors 100 for an endoscope are inserted into the forceps hole of the endoscope and advance toward biological tissue, the scissor pieces 12 and 22 including the tip end bent portions 60 are prevented from interfering with the wall surface of the forceps hole.

As illustrated in FIG. 8A, in a far base end portion 67 accommodated inside the holding frame 20, the scissor pieces 12 and 22 are parallel to the advance/retreat portion 26 and are disposed along the extension line L of the operation wire 30. A part of such a far base end portion 67 protrudes to the tip end side beyond the holding frame 20. The base end bent portions 62 are provided so as to be connected to the tip end side of the far base end portion 67.

The plurality of concave portions 15 are formed in at least any of the cutting portions 13 and 23 of the one or the other of the pair of scissor pieces 12 and 22 so as to be arranged while being separated from each other in the tip end - base end direction. In the present embodiment, with respect to both the cutting portions 13 and 23 of the pair of scissor pieces 12 and 22, the concave portions 15 (15a to 15d) and 25 of four each are formed at mutually corresponding positions. The concave portions 15 and 25 are formed on the base end side beyond the intermediate portion of the scissor pieces 12 and 22 in the tip end - base end direction. Specifically, the concave portion 15 is formed in the base end bent portion 62 of the cutting portion 13, and the concave portion 25 is formed in the base end bent portion 62 of the cutting portion 23. The remaining length region excluding the forming region of the concave portion 15 in the cutting portion 13 is formed so as to have a continuously linear shape. Similarly, the remaining length region excluding the forming region of the concave portion 25 in the cutting portion 23 is formed so as to have a continuously linear shape. That is, the cutting portions 13 and 23 are formed so as to have flat and linear shapes at the tip end bent portions 60 (however, excluding the claw portion 14).

When the tip end bent portions 60 are bent-formed in the scissor pieces 12 and 22 on the tip end side beyond the length region of the concave portions 15 and 25, in a state where the concave portions 15 and 25 intrude into the mucosal layer 202 and the biological tissue 200 is gripped by the tip end treatment portion 10 (refer to FIG. 5), the mucosal layer 202 or the submucosal layer 204 can be incised shallow by the tip end bent portions 60. Accordingly, incising the muscle layer 206 by the tip end treatment portion 10 is preferably avoided.

In the tip end treatment portion 10 of the present embodiment, spacer portions 19 are disposed between the scissor piece 12 and the holding frame 20 and between the scissor piece 22 and the holding frame 20. The spacer portions 19 have annular shapes, and the turning pivot 29 is inserted through the spacer portions 19. The spacer portions 19 are fixedly provided on the outer surfaces of the scissor pieces 12 and 22 or the inner surfaces of the holding frame 20. In this manner, when the spacer portions 19 are provided in the tip end treatment portion 10, during an opening-closing operation of the scissors 100 for an endoscope or assembly work, contact between the outer surfaces of the scissor pieces 12 and 22 and the inner surfaces of the holding frame 20 is avoided. Therefore, the insulation coats 18 and 28 formed on the outer surfaces of the scissor pieces 12 and 22 are prevented from peeling off due to unexpected contact with the holding frame 20.

The present invention is not limited to the embodiments described above and includes various aspects such as modifications and improvements as long as the object of the present invention is achieved.

For example, in the above-described embodiments, an example in which the sheath 40 is formed with a coil made of a conductive wire is illustrated. However, the embodiments are not limited thereto. In addition, in the above-described embodiment, the monopolar-type scissors 100 for an endoscope applying a high frequency voltage having the coordinate phase to the scissor pieces 12 and 22 is exemplified. However, the embodiments are not limited thereto. A bipolar-type scissors 100 for an endoscope, in which one of the scissor pieces 12 and 22 serves as an active electrode and the other serves as a return electrode may be adopted.

The various types of configuration elements in the scissors 100 for an endoscope of the present invention are not necessarily present in an individually independent manner. It is allowed to have a plurality of configuration elements to be formed as one member, to have one configuration element to be formed as a plurality of members, to have a certain configuration element to be a part of a different configuration element, to have a part of a certain configuration element and a part of a different configuration element to overlap each other, and the like.

Subsequently, an endoscopic high frequency treatment tool of the present invention will be described. The various types of configuration elements in the endoscopic high frequency treatment tool of the present invention are not necessarily present in an individually independent manner. It is allowed to have a plurality of configuration elements to be formed as one member, to have one configuration element to be formed as a plurality of members, to have a certain configuration element to be a part of a different configuration element, to have a part of a certain configuration element and a part of a different configuration element to overlap each other, and the like.

In regard to the following embodiments, the tip end denotes an insertion tip end when the endoscopic high frequency treatment tool of the present invention is inserted into the forceps hole of the endoscope, and the base end denotes an end portion on a side opposite to the tip end, that is, an end portion on the operator side.

In addition, in regard to the following the embodiments, the pair of gripping pieces denotes a portion which can be operated through an operation of an operation portion so as to be opened and closed, and denotes a portion which can interpose biological tissue by being operated in the closing direction. For example, the gripping pieces in the present invention include scissor pieces provided with thin plate-shaped cutting portions, and bowl-shaped cup pieces of which opening portions face each other.

In addition, in the following the embodiments, in a case where unless otherwise specified, the expression "in a side view" denotes that arm portions 1016 are viewed in a pivot center direction of a turning pivot 1029.

### <Fifth Embodiment>

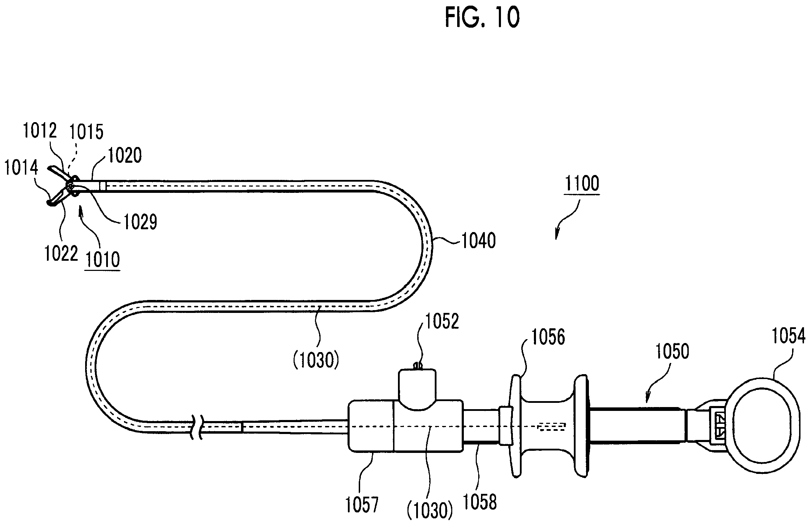

Hereinafter, a high frequency treatment tool 1100 for an endoscope according to a fifth embodiment of the present invention will be described by using FIGS. 10 to 13B. In addition, for comparison, an endoscopic high frequency treatment tool as the technology in the related art is illustrated in FIGS. 15 and 16.

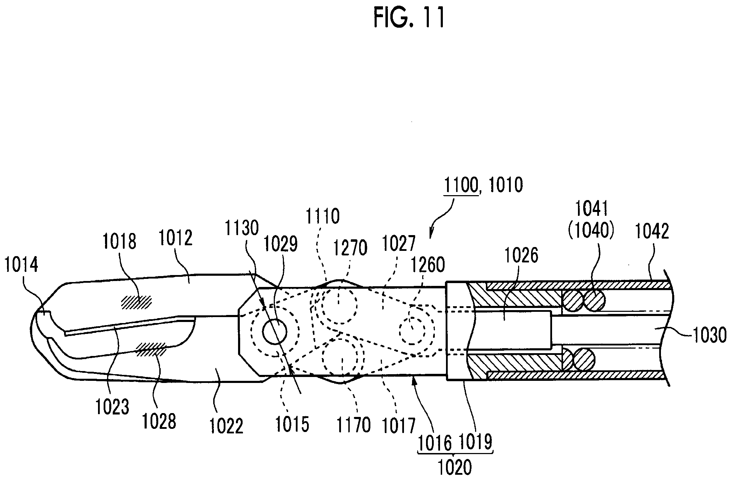

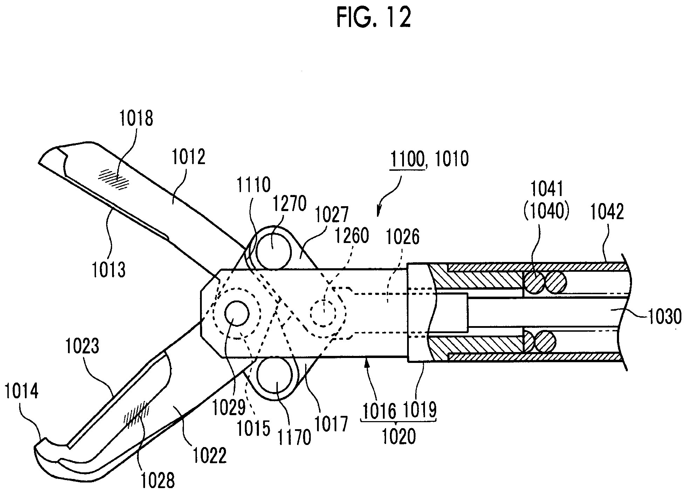

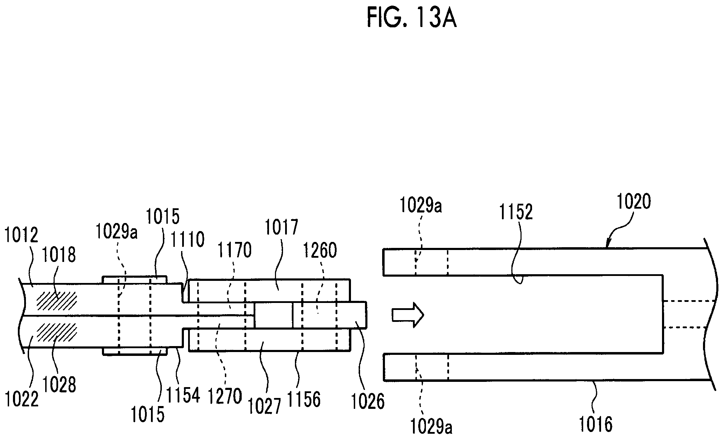

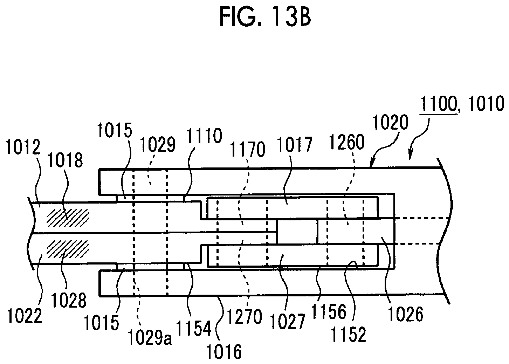

FIG. 10 is an overall side view of the high frequency treatment tool 1100 for an endoscope according to the fifth embodiment of the present invention. FIG. 11 is a side view of a tip end portion of the high frequency treatment tool 1100 for an endoscope in a closed state. FIG. 12 is a side view of the tip end portion of the high frequency treatment tool 1100 for an endoscope in a released state. FIG. 13A is a partial top view of a treatment portion 1010 and a holding frame 1020 before being assembled, and FIG. 13B is a partial top view of the treatment portion 1010 and the holding frame 1020 of the high frequency treatment tool 1100 for an endoscope.

FIG. 15 is a side view of a tip end portion of a high frequency treatment tool 1900 for an endoscope in the related art and illustrates a pair of scissor pieces 1212 and 1222 in a released state. FIG. 16 is a side view of the tip end portion of the high frequency treatment tool 1900 for an endoscope in the related art and illustrates the pair of scissor pieces 1212 and 1222 in a closed state.

In FIGS. 11, 12, 14A, 14B, and 15, in order to make understanding easy, coils 1041, insulation coats 1042, and a base end portion 1019 are illustrated in longitudinal sections.

First, the outline of the high frequency treatment tool 1100 for an endoscope of the present embodiment will be described.

The high frequency treatment tool 1100 for an endoscope has an operation wire 1030, the treatment portion 1010, a connection terminal 1052, an operation portion 1050, and the holding frame 1020.

The operation wire 1030 is a conductive wire which is inserted through a flexible tube (sheath 1040) inserted into a human body, so as to freely advance and retreat.

The treatment portion 1010 includes gripping pieces (scissor pieces 1012 and 1022). The gripping pieces (scissor pieces 1012 and 1022) are pivotally supported by the turning pivot 1029 so as to be able to mutually perform an opening-closing operation and include conductive cutting portions 1013 and 1023. Substantially the entire surfaces thereof on peripheral surfaces excluding the cutting portions 1013 and 1023 are coated with insulation coats 1018 and 1028. The connection terminal 1052 is a terminal which allows a high frequency voltage to be applied to the cutting portions 1013 and 1023.

The operation portion 1050 is installed in the base end portion of the flexible tube (sheath 1040), operates the treatment portion 1010 by performing an advance/retreat operation through the operation wire 1030, and opens and closes the gripping pieces (scissor pieces 1012 and 1022). The holding frame 1020 partially accommodates the treatment portion 1010 on the inner side between a pair of arm portions 1016 facing each other and holds both ends of the turning pivot 1029.

The high frequency treatment tool 1100 for an endoscope further includes a spacer portion 1015 which is disposed between an inner surface 1152 (refer to FIGS. 13A and 13B) of the arm portion 1016 and a counter-facing surface 1154 (refer to FIGS. 13A and 13B) facing the inner surface 1152 of the arm portion 1016 in the treatment portion 1010 accommodated between the arm portions 1016, and causes the inner surface 1152 of the arm portion 1016 and the counter-facing surface 1154 of the treatment portion 1010 to be separated from each other.

The high frequency treatment tool 1100 for an endoscope is held in the holding frame 1020 in a state where the treatment portion 1010 including the gripping pieces (scissor pieces 1012 and 1022) is accommodated on the inner side between the pair of arm portions 1016. In the high frequency treatment tool 1100 for an endoscope, since the spacer portions 1015 are disposed, direct contact between the inner surfaces 1152 of the arm portions 1016 and the counter-facing surfaces 1154 of the treatment portion 1010 is avoided. Therefore, there is no abrasion between the inner surfaces 1152 of the arm portions 1016 and the counter-facing surfaces 1154 of the treatment portion 1010 caused through an opening-closing operation of the gripping pieces (scissor pieces 1012 and 1022), and the insulation coats 1018 and 1028 provided on the outer surfaces of the counter-facing surface 1154 do not peel off.

The treatment portion 1010 in the present embodiment has the scissor pieces 1012 and 1022 which serve as the gripping pieces, and link pieces 1017 and 1027 which are interlocked with the scissor pieces 1012 and 1022. Insulation coats (not illustrated) are formed on the outer surfaces of the link pieces 1017 and 1027 as well. Since the spacer portions 1015 are disposed, the counter-facing surface 1154 (refer to FIGS. 13A and 13B) facing the inner surfaces 1152 of the arm portions 1016 of the link pieces 1017 and 1027, and the inner surfaces 1152 of the arm portions 1016 are separated from each other. Therefore, even if the gripping pieces (scissor pieces 1012 and 1022) perform an opening-closing operation, there is no abrasion and the insulation coats formed on the outer surfaces of the link pieces 1017 and 1027 do not peel off.

Therefore, in the high frequency treatment tool 1100 for an endoscope, the insulation coats on the outside surfaces of the treatment portion 1010 are prevented from peeling off, and the regions exposed to the outside of the arm portions 1016 are prevented from being conductive due to an opening-closing operation of the gripping pieces (scissor pieces 1012 and 1022).

As illustrated in FIGS. 13A and 13B, the spacer portions 1015 in the present embodiment are interposed between the counter-facing surfaces 1154 of the scissor pieces 1012 and 1022 serving as the gripping pieces accommodated in the arm portions 1016 and the inner surfaces 1152 of the arm portions 1016 with no gap. Here, the state with no gap denotes that at least parts of the inner surface 1152, the spacer portion 1015, and the counter-facing surface 1154 are continuously provided to the extent that the scissor pieces 1012 and 1022 are not shifted inside the arm portions 1016 in the pivot center direction. Therefore, such a configuration does not rule out an aspect of having a microscopic gap between the inner surface 1152 and the spacer portion 1015 or between the spacer portion 1015 and the counter-facing surface 1154.

In the high frequency treatment tool 1100 for an endoscope having such a configuration, disposition positions of the scissor pieces 1012 and 1022 accommodated in the arm portions 1016 are not shifted in the pivot center direction of the turning pivot 1029, and the disposition positions of the scissor pieces 1012 and 1022 are stable. Since the disposition positions are stable, the operability of the scissor pieces 1012 and 1022 is favorable.

In regard to the present embodiment described below, description will be given by using the high frequency treatment tool 1100 for an endoscope having the scissor pieces 1012 and 1022 as the gripping pieces.

That is, in the high frequency treatment tool 1100 for an endoscope, the pair of gripping pieces is the pair of scissor pieces 1012 and 1022 respectively having the thin plate-shaped cutting portions 1013 and 1023. Each of cutting edges of the thin plate-shaped cutting portions 1013 and 1023 is oriented toward the closing direction and applies a shearing force to biological tissue (not illustrated) through an operation of the pair of scissor pieces 1012 and 1022 in the closing direction. In a state where a shearing force is applied to the biological tissue with the cutting portions 1013 and 1023, when a high frequency voltage is applied to the cutting portions 1013 and 1023, the biological tissue can be cauterized and incised (excised).

The pair of scissor pieces 1012 and 1022 in the present embodiment is pivotally supported by the turning pivot 1029 in a region of the intermediate portion in which scissor pieces 1012 and 1022 overlap each other. The cutting portions 1013 and 1023 in the scissor pieces 1012 and 1022 are operated in a direction of overlapping each other (closing direction) while having a place of being pivotally supported by the turning pivot 1029, as a fulcrum, and thus, the biological tissue can be interposed therebetween in a state close to a closed state or a closed state. Since the relative positional relationship is stable, the cutting portions 1013 and 1023 can favorably interpose the biological tissue therebetween. Therefore, particularly in the present embodiment in which the gripping pieces are the scissor pieces 1012 and 1022, as described above, it is preferable that the spacer portions 1015 are interposed between the counter-facing surfaces 1154 in the scissor pieces 1012 and 1022 and the inner surfaces 1152 in the arm portions 1016 with no gap and the disposition position of the scissor piece 1012 is stable.

Subsequently, the high frequency treatment tool 1100 for an endoscope of the present embodiment will be described in detail.

The high frequency treatment tool 1100 for an endoscope is an instrument which is used by being inserted into the forceps hole (not illustrated) of the endoscope, applies a shearing force to the biological tissue (not illustrated), and applies a high frequency voltage, thereby performing an incision. The high frequency treatment tool 1100 for an endoscope includes the pair of scissor pieces 1012 and 1022 and the operation wire 1030. The pair of scissor pieces 1012 and 1022 are respectively provided with the cutting portions 1013 and 1023 which are pivotally supported so as to be able to be mutually opened and closed, and apply a shearing force to biological tissue. The treatment portion 1010 including the pair of scissor pieces 1012 and 1022 is provided at the tip end portion of the high frequency treatment tool 1100 for an endoscope. The treatment portion 1010 is driven through the operation wire 1030 so as to be opened and closed. For example, the operation wire 1030 is made of a conductive metal material such as stainless steel. The operation wire 1030 is a member which allows a driving force to be applied to the base end portion sides of the scissor pieces 1012 and 1022 and to perform an opening-closing operation of the tip end portions of the scissor pieces 1012 and 1022.

In the scissor pieces 1012 and 1022, the cutting portions 1013 and 1023 have thin plate shapes, and regions other than the cutting portions 1013 and 1023 have thin plate shapes having thicknesses slightly greater than that of the cutting portion 1023. The scissor pieces 1012 and 1022 overlap each other in the intermediate portion and are pivotally supported by the turning pivot 1029 so as to be able to be opened and closed. The turning pivot 1029 is a fixed pivot which is held by the holding frame 1020 and is fixed. By using the operation wire 1030, an operation is performed in a direction of increasing the distance between the cutting portions 1013 and 1023 respectively provided in the pair of scissor pieces 1012 and 1022, and the scissor pieces 1012 and 1022 are turned in the opening direction. Accordingly, the pair of scissor pieces 1012 and 1022 is in a released state. Meanwhile, through an operation of the operation portion 1050, an operation is performed in a direction of decreasing the distance between the cutting portions 1013 and 1023 respectively provided in the pair of scissor pieces 1012 and 1022, and the scissor pieces 1012 and 1022 are turned in the closing direction. Accordingly, the pair of scissor pieces 1012 and 1022 is in a closed state.

As illustrated in FIGS. 10 to 12, the holding frame 1020 is mounted in the tip end of the sheath 1040. The holding frame 1020 has the base end portion 1019 and the arm portions 1016. The base end portion 1019 is inserted into the sheath 1040 on the tip end side. Accordingly, the holding frame 1020 is mounted so as to be fixed to the tip end of the sheath 1040. The holding frame 1020 has two arm portions 1016 which protrude in a direction toward the tip end of the high frequency treatment tool 1100 for an endoscope from the base end portion 1019 and face each other. The two arm portions 1016 face each other over a predetermined space therebetween. Between the two arm portions 1016, the treatment portion 1010 is accommodated, and the turning pivot 1029 extends across the two arm portions 1016. Both ends of the turning pivot 1029 are held by the two arm portions 1016. Accordingly, the turning pivot 1029 serves as the fixed pivot of which the position is relatively fixed to the high frequency treatment tool 1100 for an endoscope.

The holding frame 1020 may be formed of a metal material which is the same as or different from those of the scissor pieces 1012 and 1022, and the insulation coats may be formed on the outer surfaces. Otherwise, the holding frame 1020 may be formed of an insulation material such as ceramic and a resin material.

As illustrated in FIGS. 10 to 13B, the high frequency treatment tool 1100 for an endoscope has the spacer portions 1015. As illustrated in FIG. 13B, in the high frequency treatment tool 1100 for an endoscope, two spacer portions 1015 are disposed so as to face each other while interposing the treatment portion 1010 therebetween. Therefore, the disposed state of the treatment portion 1010 is stable on the inner side between the arm portions 1016.

The spacer portions 1015 in the present embodiment are completely accommodated in the holding frame 1020, and in a side view, the spacer portions 1015 in their entirety are covered by the two arm portions 1016.

The spacer portion 1015 may be formed of a metal material which is the same as or different from those of the scissor pieces 1012 and 1022, and the insulation coats may be formed on the outer surfaces. Otherwise, the holding frame 1020 may be formed of an insulation material such as ceramic and a resin material.

As illustrated in FIG. 13B, in the high frequency treatment tool 1100 for an endoscope, the two spacer portions 1015 are disposed so as to face each other while interposing the pair of scissor pieces 1012 and 1022 therebetween. The scissor pieces 1012 and 1022 are continuously connected to the arm portions 1016 via the spacer portions 1015. Therefore, the disposed state of the scissor pieces 1012 and 1022 is stable on the inner side between the arm portions 1016. The state of being continuously connected mentioned herein denotes that the scissor pieces 1012 and 1022 and the spacer portions 1015, and the spacer portions 1015 and the arm portions 1016 are adjacent to each other to the extent that the disposed state of the scissor pieces 1012 and 1022 is stable between the arm portions 1016 facing each other.

In the present embodiment, the spacer portions 1015 are provided on the periphery of the turning pivot 1029. Therefore, the disposition stability of the pair of scissor pieces 1012 and 1022 pivotally supported by the turning pivot 1029 is favorable.

That is, the spacer portions 1015 in the present embodiment may be provided in places other than the periphery of the turning pivot 1029, for example, on counter-facing surfaces 1156 (refer to FIGS. 13A and 13B) in link pieces 1017. However, in such a configuration, even though the scissor pieces 1012 and 1022 and the arm portions 1016 are separated from each other, when the scissor pieces 1012 and 1022 are operated through an operation of the operation portion 1050, there is a possibility that the scissor pieces 1012 and 1022 are slightly shifted with respect to a direction of the arm portions 1016. In contrast, in an aspect in which the spacer portions 1015 are provided on the counter-facing surfaces 1154 (refer to FIGS. 13A and 13B) in the scissor pieces 1012 and 1022, particularly in an aspect in which the spacer portions 1015 are provided on the periphery of the turning pivot 1029, the above-described possibility of shifting of the scissor pieces 1012 and 1022 is remarkably reduced. The reason is that the scissor pieces 1012 and 1022 accommodated in the holding frame 1020 are practically and continuously connected to the inner surfaces 1152 of the arm portions 1016 via the spacer portions 1015.

The spacer portions 1015 in the high frequency treatment tool 1100 for an endoscope are plane plate-shaped bodies and has a hole 1029a (refer to FIG. 13A) penetrating the spacer portions 1015 in the thickness direction, and the turning pivot 1029 is inserted through the hole 1029a (refer to FIG. 13B). That is, the spacer portions 1015 are continuously provided on the periphery of the turning pivot 1029.

When the pair of scissor pieces 1012 and 1022 is operated in an opening-closing direction through an operation of the operation portion 1050, a uniform load is applied to the turning pivot 1029 which is the fixed pivot. Therefore, when the spacer portions 1015 having substantially uniform thicknesses are provided between the arm portions 1016 and the scissor pieces 1012 and 1022 separated from each other, that is, at positions continuously surrounding the periphery of the turning pivot 1029, the load of the turning pivot 1029 can be reduced and the turning pivot 1029 can be protected.

More specifically, the spacer portions 1015 in the high frequency treatment tool 1100 for an endoscope have disk shapes and are provided with the hole 1029a substantially at the center thereof.

The shapes of the spacer portions 1015 are not particularly limited. However, by having the disk shapes, rotational symmetry can be applied to the spacer portions 1015. Accordingly, the area of the contact region between the pair of scissor pieces 1012 and 1022 and the spacer portions 1015 performing an opening-closing operation in a rotary direction while having the turning pivot 1029 as the center can be minimally restrained.

In addition, for example, in the aspect in which the spacer portions 1015 are fixed to the scissor pieces 1012 and 1022, when the spacer portions 1015 have the rotational symmetry, under an opening-closing operation of the pair of scissor pieces 1012 and 1022 in the rotary direction, the spacer portions 1015 are unlikely to come to the surface out of the arm portions 1016.

Here, the disk shape includes all of a cylindrical shape having substantially the same diameter from one surface to the other surface, and a tapered shape having a diameter continuously or discontinuously reduced from one surface toward the other surface. In a case where the spacer portions 1015 have the tapered shapes and the areas are different from each other on one surface and the other surface, it is preferable that the surface having the greater area is caused to be the fixed surface which is fixed to any of the arm portions 1016 or the scissor pieces 1012 and 1022, and the surface having the smaller area is caused to be the non-fixed surface. The reason is that the contact area between the spacer portions 1015 and other members can be further reduced inside the holding frame 1020 at the time of an opening-closing operation of the pair of scissor pieces 1012 and 1022.

The outer diameters of the disk-shaped spacer portions 1015 can be equal to or greater than half a width size 1130 (refer to FIG. 11) of the place which is penetrated by the turning pivot 1029 of the gripping pieces (scissor pieces 1012 and 1022).

In this manner, with respect to the width size 1130 of the scissor piece 1012, when the spacer portions 1015 having sufficiently significant shapes are provided, not only the scissor pieces 1012 and 1022 and the arm portions 1016 are simply separated from each other, but also the inner surfaces 1152 of the arm portions 1016 and the spacer portions 1015 can come into surface contact with each other in sufficient areas. Therefore, oscillation of the scissor pieces 1012 and 1022 in the thickness direction is restricted, and thus, the disposition stability of the scissor pieces 1012 and 1022 separated from the arm portions 1016 can be favorably realized.

As illustrated in FIGS. 13A and 13B, in each of the spacer portions 1015, the scissor pieces 1012 and 1022, and the two arm portions 1016 facing each other in the present embodiment, the hole 1029a is formed. The correctly disposed hole 1029a in each configuration is adjusted in one direction, thereby aligning a series of holes 1029a through which the turning pivot 1029 is inserted. Both ends of the turning pivot 1029 (not illustrated) are fixed to the intermediate portion of the arm portions 1016 in the thickness direction or the outside surfaces.

As illustrated in FIG. 13A, the spacer portions 1015 in the present embodiment are fixed to the outer peripheral surfaces (counter-facing surfaces 1154) of the gripping pieces (scissor pieces 1012 and 1022). According to such a configuration, the high frequency treatment tool 1100 for an endoscope can exhibit the following effects.

Under an opening-closing operation of the pair of scissor pieces 1012 and 1022, the spacer portions 1015 are not chafed with the scissor pieces 1012 and 1022. Therefore, in the scissor pieces 1012 and 1022 performing an opening-closing operation, there is no possibility that the insulation coats 1018 and 1028 provided on the outer surfaces of the scissor pieces 1012 and 1022 peel off due to contact with the spacer portion 1015. The spacer portions 1015 fixed to the scissor pieces 1012 and 1022 come into contact with the inner surfaces 1152 of the arm portions 1016 under an opening-closing operation of the pair of scissor pieces 1012 and 1022. However, since the inner surfaces 1152 of the arm portions 1016 do not come into contact with biological tissue, even if a part of the insulation coats provided on the inner surfaces 1152 peels off, there is no possibility that unscheduled places in the biological tissue are cauterized. Naturally, the inner surfaces 1152 may be configured with an insulation material in advance.

Means for fixing the spacer portions 1015 to the outer peripheral surfaces (counter-facing surfaces 1154) of the scissor pieces 1012 and 1022 is not particularly limited. However, for example, by using the spacer portions 1015 formed as bodies separated from the scissor pieces 1012 and 1022, the spacer portions 1015 may be fixed to predetermined places through arbitrary means such as using a binding material and performing laser welding. Otherwise, the scissor pieces 1012 and 1022 and the spacer portions 1015 may be integrally formed of the same material such that the spacer portions 1015 may be fixed to the outer peripheral surfaces (counter-facing surface 1154) of the scissor pieces 1012 and 1022.

In the high frequency treatment tool 1100 for an endoscope, in which the spacer portions 1015 are fixed to the scissor pieces 1012 and 1022, it is preferable to pay attention to the following points.

That is, in the high frequency treatment tool 1100 for an endoscope, it is preferable that the spacer portions 1015 are included on the inner side within the outer edges of the arm portions 1016 viewed in the pivot center direction of the turning pivot 1029 (that is, in a side view) under an opening-closing operation of the pair of gripping pieces (scissor pieces 1012 and 1022).

Particularly, such a configuration is particularly effective in an aspect in which the insulation coats are provided on the outer surfaces of the spacer portions 1015 formed of conductive materials. Under an opening-closing operation of the pair of scissor pieces 1212 and 1222, even in a case where the spacer portions 1015 and the arm portions 1016 are chafed with each other and the insulation coats of the spacer portions 1015 peel off, the spacer portions 1015 including the peeling place do not come to the surface out of the arm portions 1016, and thus, there is not possibility that biological tissue is cauterized.

The high frequency treatment tool 1100 for an endoscope, in which the spacer portions 1015 are fixed to the scissor pieces 1012 and 1022 further has the following configurations.

That is, as illustrated in FIGS. 13A and 13B, in the high frequency treatment tool 1100 for an endoscope, the two spacer portions 1015 which are plane plate-shaped bodies are disposed so as to face each other while interposing the pair of gripping pieces (scissor pieces 1012 and 1022) therebetween. Here, the maximum size of the treatment portion in the pivot center direction of the turning pivot 1029 is configured to be smaller than the counter-facing width size of the spacer portions 1015.

More specifically, as illustrated in FIG. 13A, on the counter-facing surfaces 1154 of the scissor pieces 1012 and 1022, the spacer portions 1015 are respectively disposed at positions so as to face each other and are fixed. Therefore, the spacer portions 1015 serve as the protrusion portions protruding from the counter-facing surfaces 1154. On the base end sides beyond the places pivotally supported by the turning pivot 1029 of the scissor pieces 1012 and 1022, there are provided step portions 1110, and the counter-facing surfaces 1154 of the scissor pieces 1012 and 1022 are lowered inward as much as a predetermined height. The scissor pieces 1012 and 1022 have substantially uniform thickness sizes on the tip end sides beyond the step portions 1110 and have substantially uniform thickness sizes on the rear end sides beyond the step portions 1110. On the base end sides beyond the step portions 1110, the scissor pieces 1012 and 1022 are respectively interlocked with the link pieces 1017 and 1027 by pivots 1170 and 1270. The thicknesses of the link pieces 1017 and 1027 are approximately the same as the height sizes of the step portions 1110 (in FIGS. 13A and 13B, the thicknesses slightly exceed the height sizes of the step portions 1110) and are smaller than the sum of the heights of the step portions 1110 and the thicknesses of the spacer portions 1015. According to such a configuration, here, the maximum size of the treatment portion in the pivot center direction of the turning pivot 1029 is configured to be smaller than the counter-facing width sizes of the two spacer portions 1015 facing each other. Meanwhile, the distance between the inner surfaces 1152 of the arm portions 1016 facing each other is substantially equal to the counter-facing width sizes of the spacer portions 1015 facing each other.

Such a configuration exhibits advantageous effects as follows when the high frequency treatment tool 1100 for an endoscope is manufactured. That is, as illustrated in FIG. 13A, when the treatment portion 1010 is inserted from the tip end side of the two arm portions 1016 in a depth direction and is assembled by being fitted to the holding frame 1020, contact of the outside surfaces of the treatment portion 1010 with respect to the inner surfaces 1152 of the arm portions 1016 can be avoided. Accordingly, at the time of the assembly, the insulation coats provided on the outside surfaces of the treatment portion 1010 can be prevented from coming into contact with the arm portions 1016 and peeling off.

As illustrated in FIG. 10, the high frequency treatment tool 1100 for an endoscope includes the flexible sheath 1040 and the operation portion 1050 which is provided on the proximal side of the sheath 1040. The sheath 1040 is an insulation flexible tube, and the operation wire 1030 is inserted through the inside thereof.

The operation portion 1050 includes a shaft portion 1058 through which the operation wire 1030 is inserted, a finger hook ring 1054 which is provided in the base end portion of the shaft portion 1058, and a slider 1056 with which a base end of the operation wire 1030 is interlocked and which advances and retreats with respect to the shaft portion 1058. The operation wire 1030 is inserted through so as to be slidable with respect to the shaft portion 1058. For example, a user inserts a thumb into the finger hook ring 1054, and the slider 1056 is driven with two other fingers so as to advance and retreat along the longitudinal direction of the shaft portion 1058. Accordingly, the operation wire 1030 advances and retreat with respect to the operation portion 1050. The operation wire 1030 is inserted through so as to be able to advance and retreat with respect to the sheath 1040. In association with a movement of the slider 1056, the tip end portion of the operation wire 1030 advances or retreats with respect to the sheath 1040.

As illustrated in FIGS. 11 and 12, on a tip end side of the operation wire 1030, an advance/retreat portion 1026 is integrally interlocked. The advance/retreat portion 1026 and two link pieces 1017 and 1027 are mutually and pivotally supported by a pivot 1260 and are turnably interlocked with each other. Moreover, the link pieces 1017 and the scissor piece 1012 on one side are mutually and pivotally supported by a pivot 1170 and are turnably interlocked with each other. The link piece 1027 and scissor piece 1022 on the other side are mutually and pivotally supported by a pivot 1270 and are turnably interlocked with each other. The scissor piece 1012, the scissor piece 1022 on the other side, and the link pieces 1017 and 1027 relatively turn within a plane illustrated in FIGS. 11 and 12. The pivots 1170 and 1270 and the pivot 1260 are not held with respect to the holding frame 1020, and positions thereof move in association with an operation of the operation wire 1030. That is, the pivots 1170 and 1270 and the pivot 1260 are moving pivots of which the positions are relatively movable with respect to the high frequency treatment tool 1100 for an endoscope.

As illustrated in FIG. 11, when the operation wire 1030 and the advance/retreat portion 1026 are drawn to the base end side (to the right in, FIG. 11), the link pieces 1017 and 1027 and the scissor pieces 1012 and 1022 have substantially linear shapes, and the pair of scissor pieces 1012 and 1022 is in a closed state. Meanwhile, as illustrated in FIG. 12, when the operation wire 1030 and the advance/retreat portion 1026 are pushed out to the tip end side (to the left, in FIG. 12), the pivots 1170 and 1270 move in the outer direction of the holding frame 1020. Therefore, a portion in which the link pieces 1017 and 1027 and the scissor pieces 1012 and 1022 are interlocked with each other is curved, and the cutting portions 1013 and 1023 are operated in the opening direction. Accordingly, the pair of scissor pieces 1012 and 1022 is in a released state.

In the vicinity of the turning pivot 1029, the scissor pieces 1012 and 1022 have substantial L-shapes obtusely curved in the in-turning plane direction, that is, sickle shapes. The shapes of the scissor pieces 1012 and 1022 forming a pair may be symmetric or asymmetric. The scissor pieces 1012 and 1022 and the link pieces 1017 and 1027 configure a four-bar link mechanism by which the treatment portion 1010 is driven so as to be opened and closed while having the turning pivot 1029 as a fulcrum. The operation wire 1030 allows a driving force to be applied to the base end portions of the scissor pieces 1012 and 1022 via the advance/retreat portion 1026 and the link pieces 1017 and 1027, and an opening-closing operation of the tip end portions of the scissor pieces 1012 and 1022 is performed.

In this manner, as the pair of scissor pieces 1012 and 1022 switches between a closed state and a released state, the treatment portion 1010 including the scissor pieces 1012 and 1022 and the link pieces 1017 and 1027 relatively moves with respect to the holding frame 1020. When such relative movements are repeated, a portion of the high frequency treatment tool 1100 for an endoscope accommodated between the arm portions 1016 of the treatment portion 1010 comes to the surface, and a portion which has come to the surface is accommodated between the arm portions 1016, repetitively. Here, the high frequency treatment tool 1100 for an endoscope includes the above-described spacer portions 1015. Therefore, even if the treatment portion 1010 relatively moves with respect to the holding frame 1020, the inner surfaces 1152 of the arm portions 1016 and the counter-facing surfaces 1154 of the treatment portion 1010 are not chafed with each other, and the insulation coats of the counter-facing surfaces 1154 of the treatment portion 1010 do not peel off.

The sheath 1040 of the present embodiment is the coil 1041 made by tightly winding a conductive wire such as a stainless steel wire. On the outside surface of the sheath 1040, the insulation coat 1042 is tightly provided. The operation wire 1030 is connected to a rotation operation portion 1057 (refer to FIG. 10) while having friction therewith. When the rotation operation portion 1057 is subjected to a shaft-rotation around the shaft portion 1058, the operation wire 1030 of which the base end is fixed to the slider 1056 rotates inside the sheath 1040. Accordingly, the treatment portion 1010 can be directed in a desired orientation. The rotation operation portion 1057 is rotatably attached to the connection terminal 1052. In a state where a power supply cable (not illustrated) causing the connection terminal 1052 and a high frequency power source (not illustrated) to be interlocked with each other is suspended downward, the rotation operation portion 1057 can be operated so as to rotate around the shaft portion 1058. In place of the present embodiment, the slider 1056 may be configured to be able to be subjected to a shaft-rotation around the shaft portion 1058, and the function of the rotation operation portion 1057 may be applied to the slider 1056. That is, the slider 1056 may be configured to be driven so as to advance and retreat along the longitudinal direction of the shaft portion 1058 such that the operation wire 1030 advances and retreats and an opening-closing operation of the treatment portion 1010 is performed. In addition, the slider 1056 may be configured to be subjected to a shaft-rotation around the shaft portion 1058 such that the treatment portion 1010 rotates so as to be directed in a desired orientation.

In the scissor pieces 1012 and 1022, the cutting portions 1013 and 1023 are formed along end edges facing each other in each of the inner surfaces overlapping each other. The cutting portions 1013 and 1023 of the present embodiment are formed in a part of region on the tip end side beyond the turning pivot 1029 in the scissor pieces 1012 and 1022. The cutting portions 1013 and 1023 are sites configured to apply a shearing force to biological tissue (not illustrated). Specifically, when the slider 1056 (refer to FIG. 10) of the operation portion 1050 is drawn, and as illustrated in FIG. 11, the scissor pieces 1012 and 1022 are turned while having the turning pivot 1029 as the center such that the tip end portion is closed, a shearing force is generated between the cutting portions 1013 and 1023.

At the tip end portion of at least one of the pair of scissor pieces 1012 and 1022, a claw portion 1014 gripping the biological tissue is formed so as to protrude in the closing direction beyond the cutting portions 1013 and 1023. As illustrated in FIGS. 11 and 12, the high frequency treatment tool 1100 for an endoscope according to the present embodiment has the claw portion 1014 which is formed in the tip end portion of the scissor piece 1022 so as to protrude in the closing direction beyond the cutting portion 1023.

The pair of scissor pieces 1012 and 1022 is provided with the conductive cutting portions 1013 and 1023, and substantially the entire surfaces thereof on the peripheral surfaces excluding the cutting portions 1013 and 1023 are configured to be insulation. The high frequency treatment tool 1100 for an endoscope has the connection terminal 1052 configured to apply a high frequency voltage to the conductive cutting portions 1013 and 1023, applies a shearing force to the biological tissue while cutting the biological tissue between the cutting portions 1013 and 1023, and applies a high frequency voltage. Thus, the biological tissue can be incised. When an incision is performed, by utilizing high frequencies, the high frequency treatment tool 1100 for an endoscope can incise the biological tissue with a small force. In addition, bleeding of the incised site can be stopped at the same time. Here, in the high frequency treatment tool 1100 for an endoscope, the problem in which when the pair of scissor pieces 1012 and 1022 is operated, the outside surface of the treatment portion 1010 and the arm portions 1016 are chafed with each other, and the insulation coat of the treatment portion 1010 peels off is improved. Therefore, as in the high frequency treatment tool 1900 for an endoscope in the related art, the insulation coats 1018 and 1028 of exposed regions 1920 and 1940 do not become conductive due to peeling off. Thus, safety and operability in utilizing a high frequency voltage are favorable.

In the high frequency treatment tool 1100 for an endoscope, the slider 1056 includes the connection terminal 1052 which is a terminal connected to a high frequency voltage (not illustrated). In order to be able to incise biological tissue by utilizing high frequencies, all of the scissor pieces 1012 and 1022, the link pieces 1017 and 1027, and the advance/retreat portion 1026 configuring the treatment portion 1010 are made of conductive metal materials. In addition, as described above, the operation wire 1030 is also made of a conductive metal material. Therefore, a high frequency voltage applied to the connection terminal 1052 is applied to the scissor pieces 1012 and 1022.

As illustrated in FIGS. 11 and FIG. 12, the outer surfaces of the scissor pieces 1012 and 1022 are respectively provided with the insulation coats 1018 and 1028. For convenience, in FIGS. 11 and 12, the insulation coats 1018 and 1028 are partially illustrated. However, the insulation coats 1018 and 1028 are formed in the scissor pieces 1012 and 1022 substantially in their entirety excluding linear edge portions of the cutting portions 1013 and 1023. The cutting portions 1013 and 1023 are exposed from the insulation coats 1018 and 1028.

The insulation coats 1018 and 1028 can be formed by performing coating with insulation materials, for example, ceramic materials such as a fluororesin, diamond-like carbon (DLC), a titanium oxide-based material, and a silicon-based material. According to an examination of the inventor, it has been found that particularly when a silicon-based ceramic material is selected as the insulation material, it is possible to acquire the insulation coats which are particularly excellent in insulation, durability with respect to electrification, and adhesion. The insulation and the durability are achieved by increasing the coating thicknesses of the insulation coats. However, in a case where the coating thickness is increased, there is a disadvantage in that adhesion with respect to the scissor pieces is deteriorated. In contrast, when silicon-based ceramic is selected as the insulation material, compared to other ceramic materials, the fluororesin, and the DLC, insulation, durability with respect to electrification, and adhesion can be improved in a well-balanced state. When silicon-based ceramic is selected as the insulation material, abrasion resistance of the insulation coat is improved. Therefore, the insulation coats 1018 and 1028 are further preferably restrained from peeling off due to an opening-closing operation of the gripping pieces (scissor pieces 1012 and 1022).

In a case where the silicon-based ceramic is adopted as the insulation material, insulation coats 1018 and 1028 can be formed by paining the scissor pieces 1012 and 1022 with liquid insulation coating including polysiloxane (organo-polysiloxane). As the insulation coating, in addition to polysiloxane, an inorganic filler such as silica, and a coloring agent such as a binder resin and a pigment may be mixed with an aqueous menstruum or a solvent-based menstruum, or a hardening agent may be arbitrarily mixed.

The insulation coats 1018 and 1028 with which the outer surfaces of the pair of gripping pieces (scissor pieces 1012 and 1022) are respectively coated may be colored with colors different from each other. Specifically, it is favorable that a first pigment which is mixed into the insulation coat 1018 painted on the scissor piece 1012 and a second pigment which is mixed into the insulation coat 1028 painted on the scissor piece 1022 are made of different-type materials exhibiting colors different from each other to the extent of being able to be visually discriminated. Accordingly, under an endoscopic observation, the orientations of the front and the back of the scissor pieces 1012 and 1022 can be easily grasped.

The thicknesses of the insulation coats 1018 and 1028 are not particularly limited. However, in a case where a fluororesin is adopted as the insulation material, it is preferable that the content ranges from 20 µm to 80 µm. In a case of adopting DLC, it is preferable that the content ranges from 1 µm to 5 µm. In a case of adopting a silicon-based ceramic material, it is preferable that the content ranges from 5 µm to 40 µm.

The cutting portions 1013 and 1023 exposed from the insulation coats 1018 and 1028 serve as linear-shaped electrodes. The scissor pieces 1012 and 1022 serve as monopolar-type high-frequency electrodes when a high frequency voltage having the coordinate phase is applied from the connection terminal 1052.

When the cutting portions 1013 and 1023 are conductive electrodes, and the insulation coats 1018 and 1028 are formed in different regions in the scissor pieces 1012 and 1022, the insulation coats 1018 and 1028 can be formed as follows. That is, the scissor pieces 1012 and 1022 are coated with an insulation material in advance in their entirety. Thereafter, the coating in linear regions corresponding to the cutting portions 1013 and 1023 can be caused to peel off by performing polishing or using chemicals.

The holding frame 1020 and the spacer portions 1015 are made of metal materials at least of which the outside surfaces are coated with insulation coats, or insulation materials such as a ceramic material and a resin material. That is, a high frequency voltage applied to the connection terminal 1052 is not unexpectedly applied to the biological tissue through the holding frame 1020. Accordingly, the cauterized biological tissue is prevented from adhering to not only the scissor pieces 1012 and 1022 excluding the cutting portions 1013 and 1023 but also the holding frame 1020.

### <Sixth Embodiment>

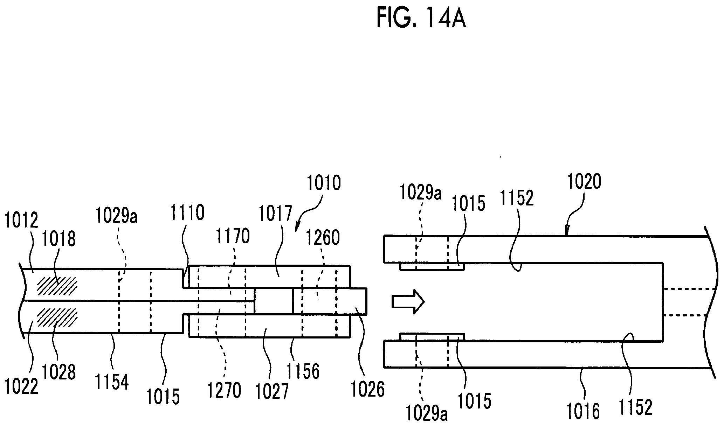

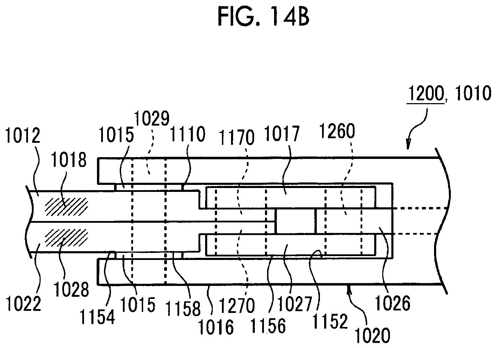

Subsequently, a high frequency treatment tool 1200 for an endoscope according to a sixth embodiment of the present invention will be described by using FIGS. 14A and 14B.

FIG. 14A is top view of the treatment portion 1010 and the holding frame 1020 in the high frequency treatment tool 1200 for an endoscope according to the sixth embodiment before being assembled, and FIG. 14B is a top view partially illustrating the treatment portion 1010 and the holding frame 1020 of the high frequency treatment tool 1200 for an endoscope, according to the sixth embodiment.

The present embodiment is different from the fifth embodiment in that in the high frequency treatment tool 1200 for an endoscope, the spacer portions 1015 are fixed to the inner surfaces of the arm portions 1016. Hereinafter, in regard to the description of the sixth embodiment, the description overlapping the fifth embodiment will be appropriately omitted.

The high frequency treatment tool 1200 for an endoscope includes the spacer portion 1015 which is disposed between the inner surface 1152 of the arm portion 1016 and the counter-facing surface 1154 facing the inner surface 1152 of the arm portion 1016 in the treatment portion 1010 accommodated between the arm portions 1016, and causes the inner surface 1152 of the arm portion 1016 and the counter-facing surface 1154 of the treatment portion 1010 to be separated from each other. Similar to the high frequency treatment tool 1100 for an endoscope, the high frequency treatment tool 1200 for an endoscope includes the scissor pieces 1012 and 1022 as the gripping pieces.

As illustrated in FIG. 14A, the spacer portions 1015 in the present embodiment are fixed to the inner surfaces 1152 of the arm portions 1016.

In regard to the spacer portions 1015 in the present embodiment, the spacer portions 1015 manufactured as bodies separated from the holding frame 1020 may be fixed to a predetermined position on the inner surfaces 1152 through arbitrary means such as bonding and laser welding, or may be integrally formed with the same material as the holding frame 1020.

For example, when a high frequency voltage is applied to the cutting portions 1013 and 1023, in a structure in which the holding frame 1020 is not included in a conductive path, the holding frame 1020 can be formed of an insulation member such as a resin. When the holding frame 1020 and the spacer portions 1015 are integrally formed of materials such as a resin having excellent integrally-forming characteristics, production efficiency can be improved.

The gripping pieces (scissor pieces 1012 and 1022) in the high frequency treatment tool 1200 for an endoscope have contact regions 1158 (refer to FIG. 14B) which come into contact with the spacer portion 1015. Trajectories of the contact regions 1158 in the gripping pieces (scissor pieces 1012 and 1022) configuring the pair of gripping pieces (scissor pieces 1012 and 1022) which are opened and closed between a released state and a closed state are included on the inner side beyond the outer edges of the arm portions 1016 viewed in the pivot center direction of the turning pivot 1029.

In response to an opening-closing operation of the pair of scissor pieces 1012 and 1022, the contact regions 1158 in the pair of scissor pieces 1012 and 1022 vary. In the contact regions 1158, since the spacer portions 1015 and the scissor pieces 1012 and 1022 are chafed with each other, there are cases where the insulation coats 1018 and 1028 of the scissor pieces 1012 and 1022 peel off. That is, the regions in which the contact regions 1158 and the insulation coats 1018 and 1028 are formed becomes substantially the same regions.

Here, when the trajectories of the contact regions 1158 are included on the inner side beyond the outer edges of the arm portions 1016 viewed in the pivot center direction of the turning pivot 1029, the regions in which the insulation coats 1018 and 1028 are formed can be prevented from coming to the surface from the holding frame 1020.

### <Seventh Embodiment>

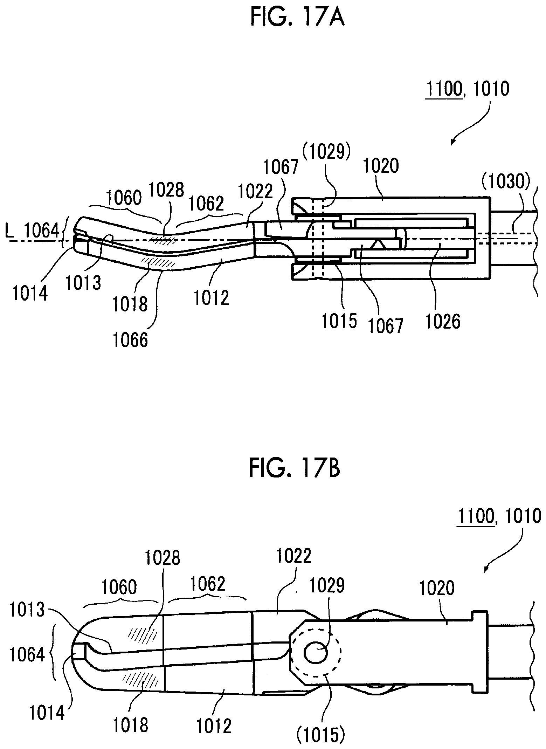

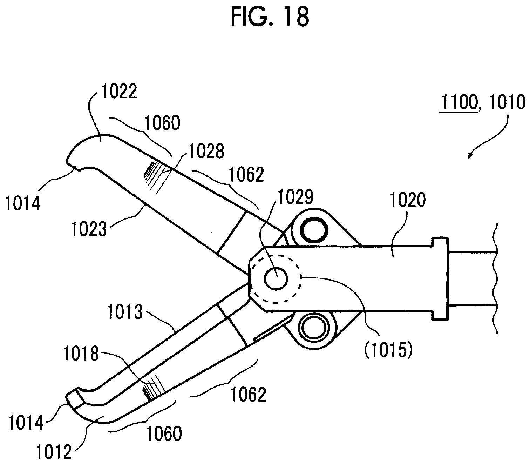

FIG. 17A is a plan view illustrating a closed state of the tip end treatment portion 1010 in scissors 1100 for an endoscope, according to a seventh embodiment, and FIG. 17B is a side view of the tip end treatment portion 1010. FIG. 18 is a side view illustrating a released state of the tip end treatment portion 1010 in the scissors 1100 for an endoscope, according to the seventh embodiment. The description overlapping the first or sixth embodiment will be appropriately omitted.

The present embodiment is different from the fifth embodiment in that the pair of scissor pieces 1012 and 1022 in the scissors 1100 for an endoscope individually has a tip end bent portion 1060 which is bent to one side in the thickness direction (upward, in FIG. 17A).

The tip end bent portion 1060 can be made by bend-forming the plate-shaped scissor pieces 1012 and 1022 in the plane-perpendicular direction. More specifically, when the intermediate portion of the scissor pieces 1012 and 1022 is subjected to bend-forming in the plane-perpendicular direction and a bent projection portion 1066 is formed, tip ends 1064 of the scissor pieces 1012 and 1022 are oriented in an oblique direction with respect to the extension line L of the operation wire 1030. The extension line L is a virtual line in the scissors 1100 for an endoscope and is illustrated with a two-dot chain line in FIG. 17A.

The bent projection portion 1066 is a convex region formed on a side (lower side, in FIG. 17A) opposite to the bending direction of the tip end bent portions 1060 in the scissor pieces 1012 and 1022. The radii of curvature of the scissor pieces 1012 and 1022 in the bent projection portion 1066 are not particularly limited. However, it is preferable that the tip end bent portions 1060 and the base end bent portions 1062 are formed so as to be smoothly continuous at the bent projection portion 1066. As in the present embodiment, since the tip end bent portions 1060 are formed, when the tip end treatment portion 1010 advances inside a body cavity, the bent projection portion 1066 abuts on the biological tissue. Therefore, the tip end 1064 of the tip end treatment portion 1010 is oriented to a side opposite to the biological tissue. Thus, the tip end 1064 is restrained from interfering with the biological tissue.

In the scissors 1100 for an endoscope of the present embodiment, the pair of scissor pieces 1012 and 1022 individually has the base end bent portion 1062 which is bent toward the other side (downward, in FIG. 17A) in the thickness direction, on the base end side beyond the tip end bent portions 1060. The tip end 1064 of the pair of scissor pieces 1012 and 1022 are positioned on the extension line L of the operation wire 1030. In addition, when the scissors 1100 for an endoscope are viewed from the tip end side, the scissor pieces 1012 and 1022 including the tip end bent portions 1060 and the base end bent portions 1062 are stored inside the envelope region of the holding frame 1020. Accordingly, when the scissors 1100 for an endoscope are inserted into the forceps hole of the endoscope and advance toward biological tissue, the scissor pieces 1012 and 1022 including the tip end bent portions 1060 are prevented from interfering with the wall surface of the forceps hole. Therefore, the insulation coats 1018 and 1028 of the tip end treatment portion 1010 are prevented from coming into frictional contact with the wall surface of the forceps hole and being worn.

As illustrated in FIG. 17A, in a far base end portion 1067 accommodated inside the holding frame 1020, the scissor pieces 1012 and 1022 are parallel to the advance/retreat portion 1026 and are disposed along the extension line L of the operation wire 1030. A part of such a far base end portion 1067 protrudes to the tip end side beyond the holding frame 1020. The base end bent portions 1062 are provided so as to be connected to the tip end side of the far base end portion 1067.

Hereinbefore, the fifth embodiment to the seventh embodiment of the present invention are described. However, the present invention is not limited to the embodiments described above and includes various aspects such as modifications and improvements as long as the object of the present invention is achieved.

In addition, in the present embodiment, an example in which the spacer portions 1015 are disposed between the scissor pieces 1012 and 1022 and the arm portions 1016 is illustrated. However, the present embodiment includes an aspect in which the spacer portions 1015 are disposed at arbitrary places in the treatment portion 1010 other than the scissor pieces 1012 and 1022 (for example, between the counter-facing surfaces 1156 (refer to FIGS. 13A and 13B) facing each other on the inner surfaces 1152 of the arm portions 1016 of the link pieces 1017 and 1027, and the inner surfaces 1152 of the arm portions 1016.

In addition, in the above-described embodiment, an example in which the spacer portions 1015 are fixed and disposed on the counter-facing surfaces 1154 in the scissor pieces 1012 and 1022, or the inner surfaces 1152 of the arm portions 1016 is illustrated. However, the aspect of the spacer portions 1015 is not limited thereto. For example, the turning pivot 1029 may penetrate the middle of the spacer portions 1015, and in a state of being not fixed to any of the treatment portion 1010 and the arm portions 1016, the spacer portions 1015 may be pinched by the treatment portion 1010 and the arm portions 1016.

In addition, in the description of the present embodiment, an example of the spacer portions 1015 that includes the hole 1029a through which the turning pivot 1029 is inserted and continuously disposed on the periphery of the turning pivot 1029 is illustrated. However, such an example does not limit the disposition positions of the spacer portions 1015. For example, a plurality of the spacer portions 1015 may be provided between the scissor piece 1012 and the arm portions 1016 so as to be dispersed on the periphery of the turning pivot 1029.

The above-described embodiments include the following technical ideas.
(1) Endoscopic scissors, configured to be inserted into a forceps hole of an endoscope and incise biological tissue. The endoscopic scissors include a pair of scissor pieces that face and overlap each other, are pivotally supported by a turning pivot extending in an overlapping direction so as to be able to be opened and closed, and respectively have cutting portions configured to grip and incise the biological tissue; and an operation wire configured to apply a driving force to base end portion sides of the scissor pieces such that an opening-closing operation of tip end portions of the scissor pieces is performed. At the tip end portion of at least one of the pair of scissor pieces, a claw portion configured to grip the biological tissue is formed so as to protrude in a closing direction beyond the cutting portion. A concave portion penetrating the cutting portion in a thickness direction is locally formed in the cutting portion of at least one of the scissor pieces, and the cutting portion excluding the claw portion and the concave portion is formed so as to have a flat and linear shape extending in a tip end - base end direction.
(2) The endoscopic scissors according to (1), in which the concave portion has a substantially semicircular shape.
(3) The endoscopic scissors according to (2), in which the concave portion having a substantially semicircular shape are respectively formed at mutually corresponding positions in the cutting portions of the pair of scissor pieces so as to face each other to form a substantially circular-shaped penetration hole, when the scissor pieces are closed and the concave portions meet together.
(4) The endoscopic scissors according to any one of (1) to (3), in which the concave portion is formed on a base end side beyond an intermediate portion in the tip end - base end direction of the scissor pieces.
(5) The endoscopic scissors according to any one of (1) to (4), in which a plurality of the concave portions are formed in any cutting portion of the at least one of scissor pieces so as to be arranged while being separated from each other in the tip end - base end direction, and a remaining cutting portion is formed so as to have a continuously linear shape.
(6) The endoscopic scissors according to any one of (1) to (5), further include a power supply portion configured to apply a high frequency voltage to the scissor pieces through the operation wire. Insulation coats are respectively provided on outer surfaces of the scissor pieces. The cutting portion and an inner peripheral surface of the concave portion are exposed from the insulation coat.
   In addition, the above-described embodiments include the following technical ideas.
(7) The endoscopic scissors, in which the pair of scissor pieces individually has a tip end bent portion which is bent toward one side in the overlapping direction, on a tip end side beyond a forming region of the concave portions.
(8) The endoscopic scissors, in which the pair of scissor pieces individually has a base end bent portion which is bent toward the other side in the overlapping direction on the base end side beyond the tip end bent portion, and the tip ends of the pair of scissor pieces are positioned on an extension line of the operation wire.
(9) The endoscopic scissors, in which a plurality of concave portions are formed in at least any cutting portion of the at least one of the pair of scissor pieces so as to be arranged while being separated from each other in the tip end - base end direction, and a remaining length region excluding the forming region of the concave portions in the cutting portion is formed so as to have a continuously linear shape.
(10) The endoscopic scissors further including a power supply portion configured to apply a high frequency voltage to the scissor pieces through the operation wire. Insulation coats are respectively provided on outer surfaces of the pair of scissor pieces. The cutting portion and an inner peripheral surface of the concave portion are exposed from the insulation coat.
(11) The endoscopic scissors, in which the insulation coat contains silicon-based ceramic.
(12) The endoscopic scissors, in which the insulation coats respectively provided on the outer surfaces of the pair of scissor pieces are colored with colors different from each other.
   Moreover, the above-described embodiments include the following technical ideas.
(13) An endoscopic high frequency treatment tool includes a conductive operation wire that is inserted through a flexible tube configured to be inserted into a human body, so as to freely advance and retreat; a treatment portion that includes a pair of gripping pieces which are pivotally supported by a turning pivot so as to be able to mutually perform an opening-closing operation, the gripping pieces including conductive cutting portions and insulation coats coating substantially entire peripheries thereof excluding the cutting portions, and the treatment portion being disposed at a tip end portion of the operation wire; a connection terminal configured to apply a high frequency voltage to the cutting portions; an operation portion that is installed in a base end portion of the flexible tube, and configured to open and close he gripping pieces by operating the treatment portion by performing an advance-retreat operation through the operation wire; a holding frame that partially accommodates the treatment portion on the inner side between a pair of arm portions facing each other and holds both ends of the turning pivot; and a spacer portion that is disposed between an inner surface of the arm portion and a counter-facing surface facing the inner surface of the arm portion in the treatment portion accommodated between the arm portions, and is configured to separate the inner surface of the arm portion and the counter-facing surface of the treatment portion from each other.
(14) The endoscopic high frequency treatment tool according to (13), in which the spacer portion is interposed between the counter-facing surface of the gripping piece accommodated between the arm portions and the inner surface of the arm portion, with no gap.
(15) The endoscopic high frequency treatment tool, according to (13) or (14), in which two of the spacer portions are disposed so as to face each other while interposing the treatment portion therebetween.
(16) The endoscopic high frequency treatment tool, according to any one of (13) to (15), in which the spacer portion is provided on the periphery of the turning pivot.
(17) The endoscopic high frequency treatment tool, according to any one of (13) to (16), in which the spacer portion is a plane plate-shaped body and has a hole penetrating the spacer portion in a thickness direction, and the turning pivot is inserted through the hole.
(18) The endoscopic high frequency treatment tool, according to (17), in which the spacer portion has a disk shape and is provided with the hole substantially at the center.
(19) The endoscopic high frequency treatment tool, according to (18), in which the outer diameter of the disk-shaped spacer portion is equal to or greater than half the width size of a place penetrated by the turning pivot of the gripping piece.
(20) The endoscopic high frequency treatment tool, according to any one of (13) to (19), in which the spacer portion is fixed to an outer peripheral surface of the gripping piece.
(21) The endoscopic high frequency treatment tool, according to (20), in which under an opening-closing operation of the pair of gripping pieces, the spacer portion is included inside of an outer edge of the arm portion viewed in a pivot center direction of the turning pivot.
(22) The endoscopic high frequency treatment tool, according to (20) or (21), in which the two spacer portions which are plane plate-shaped bodies are disposed so as to face each other while interposing the pair of gripping pieces therebetween, and the maximum size of the treatment portion in the pivot center direction of the turning pivot is smaller than the counter-facing width size of the two spacer portions facing each other.
(23) The endoscopic high frequency treatment tool, according to any one of (13) to (19), in which the spacer portion is fixed to the inner surface of the arm portion.
(24) The endoscopic high frequency treatment tool, according to (23), in which the gripping piece has a contact region which comes into contact with the spacer portion, and a trajectory of the contact region of the gripping piece configuring the pair of gripping pieces which are opened and closed so as to be in a released state and a closed state is included inside of the outer edge of the arm portion viewed in the pivot center direction of the turning pivot.
(25) The endoscopic high frequency treatment tool, according to any one of (13) to (24), in which the gripping piece is a scissor piece having a thin plate-shaped cutting portion.
   In addition, the above-described embodiments include the following technical ideas.
(26) The endoscopic high frequency treatment tool, in which the pair of scissor pieces individually has a tip end bent portion which is bent toward one side in the thickness direction.
(27) The endoscopic high frequency treatment tool, in which the pair of scissor pieces individually has a base end bent portion which is bent toward the other side in the thickness direction, on the base end side beyond the tip end bent portion, and the tip ends of the pair of scissor pieces are positioned on an extension line of the operation wire.
(28) The endoscopic high frequency treatment tool, in which the insulation coat contains silicon-based ceramic.
(29) The endoscopic high frequency treatment tool, in which the insulation coats respectively coating the outer surfaces of the pair of gripping pieces are colored with colors different from each other.

### Reference Signs List

- 10: TIP END TREATMENT PORTION
- 12: FIRST SCISSOR PIECE
- 22: SECOND SCISSOR PIECE
- 13, 23: CUTTING PORTION
- 14: CLAW PORTION
- 15, 15a to 15d, 25: CONCAVE PORTION
- 16: PENETRATION HOLE
- 17, 27: LINK PIECE
- 18, 28: INSULATION COAT
- 19: SPACER PORTION
- 20: HOLDING FRAME
- 26: ADVANCE/RETREAT PORTION
- 29: TURNING PIVOT
- 30: OPERATION WIRE
- 40: SHEATH
- 42: INSULATION COAT
- 50: HAND OPERATION PORTION
- 52: POWER SUPPLY PORTION
- 54: FINGER HOOK RING
- 56: SLIDER
- 58: SHAFT PORTION
- 60: TIP END BENT PORTION
- 62: BASE END BENT PORTION
- 64: TIP END
- 66: BENT PROJECTION PORTION
- 67: FAR BASE END PORTION
- 100: ENDOSCOPIC SCISSORS
- 200: BIOLOGICAL TISSUE
- 202: MUCOSAL LAYER
- 203: LESION TISSUE
- 204: SUBMUCOSAL LAYER
- 206: MUSCLE LAYER
- 210: SWELLING SITE
- L: EXTENSION LINE
- 1010: TREATMENT PORTION
- 1012, 1022: SCISSOR PIECE
- 1013, 1023: CUTTING PORTION
- 1014: CLAW PORTION
- 1015: SPACER PORTION
- 1016: ARM PORTION
- 1017, 1027: LINK PIECE
- 1018, 1028: INSULATION COAT
- 1019: BASE END PORTION
- 1020: HOLDING FRAME
- 1026: ADVANCE/RETREAT PORTION
- 1029: TURNING PIVOT
- 1029a: HOLE
- 1030: OPERATION WIRE
- 1040: SHEATH
- 1041: COIL
- 1042: INSULATION COAT
- 1050: OPERATION PORTION
- 1052: CONNECTION TERMINAL
- 1054: FINGER HOOK RING
- 1056: SLIDER
- 1057: ROTATION OPERATION PORTION
- 1058: SHAFT PORTION
- 1060: TIP END BENT PORTION
- 1062: BASE END BENT PORTION
- 1064: TIP END
- 1066: BENT PROJECTION PORTION
- 1067: FAR BASE END PORTION
- 1100, 1200, 1900: ENDOSCOPIC HIGH FREQUENCY TREATMENT TOOL
- 1110: STEP PORTION
- 1130: WIDTH SIZE
- 1152: INNER SURFACE
- 1154: COUNTER-FACING SURFACE
- 1156: COUNTER-FACING SURFACE
- 1158: CONTACT REGION
- 1170, 1260, 1270: PIVOT
- 1212, 1222: SCISSOR PIECE
- 1910: ARM PORTION
- 1912: BASE END PORTION
- 1914: HOLDING FRAME
- 1920, 1940: EXPOSED REGION
- L: EXTENSION LINE

## Claims

1. Endoscopic scissors, configured to be inserted into a forceps hole of an endoscope and incise a biological tissue, the scissors comprising:
a pair of scissor pieces that face and overlap each other, are pivotally supported by a turning pivot extending in an overlapping direction so as to be able to be opened and closed, and respectively have cutting portions configured to grip and incise the biological tissue; and
an operation wire configured to apply a driving force to base end portion sides of the scissor pieces such that an opening-closing operation of tip end portions of the scissor pieces is performed,
wherein at the tip end portion of at least one of the pair of scissor pieces, a claw portion configured to grip the biological tissue protrudes in a closing direction beyond the cutting portion, and
wherein a concave portion penetrating the cutting portion in a thickness direction is locally formed in the cutting portion of at least one of the scissor pieces, and the cutting portion excluding the claw portion and the concave portion is formed so as to have a flat and linear shape extending in a tip end - base end direction.

2. The endoscopic scissors according to claim 1,
wherein the concave portion has a substantially semicircular shape.

3. The endoscopic scissors according to claim 2,
wherein the concave portions having a substantially semicircular shape are respectively formed at mutually corresponding positions in the cutting portions of the pair of scissor pieces so as to face each other to form a substantially circular-shaped penetration hole when the scissor pieces are closed and the concave portions meet together.

4. The endoscopic scissors according to any one of claims 1 to 3,
wherein the concave portion is formed on a base end side beyond an intermediate portion in the tip end - base end direction of the scissor pieces.

5. The endoscopic scissors according to claim 4,
wherein the pair of scissor pieces individually has a tip end bent portion which is bent toward one side in an overlapping direction, on a tip end side beyond a forming region of the concave portion.

6. The endoscopic scissors according to claim 5,
wherein the pair of scissor pieces individually has a base end bent portion which is bent toward the other side in the overlapping direction, on the base end side beyond the tip end bent portion, and
wherein tip ends of the pair of scissor pieces are positioned on an extension line of the operation wire.

7. The endoscopic scissors according to any one of claims 1 to 6,
wherein a plurality of the concave portions are formed in at least a cutting portion of the at least one of the pair of scissor pieces so as to be arranged while being separated from each other in the tip end - base end direction, and a remaining length region excluding the forming region of the concave portions in the cutting portion has a continuously linear shape.

8. The endoscopic scissors according to any one of claims 1 to 7, further comprising:
a power supply portion configured to apply a high frequency voltage to the scissor pieces through the operation wire, and
insulation coats respectively provided on outer surfaces of the pair of scissor pieces,
wherein the cutting portion and an inner peripheral surface of the concave portion are exposed from the insulation coat.

9. The endoscopic scissors according to claim 8,
wherein the insulation coat comprises a silicon-based ceramic.

10. The endoscopic scissors according to claim 8 or 9,
wherein the insulation coats respectively provided on the outer surfaces of the pair of scissor pieces are colored with colors different from each other.

11. An endoscopic high frequency treatment tool comprising:
a conductive operation wire inserted through a flexible tube configured to be inserted into a human body, so as to freely advance and retreat;
a treatment portion comprising a pair of gripping pieces pivotally supported by a turning pivot so as to be able to mutually perform an opening-closing operation, the gripping pieces comprising conductive cutting portions and insulation coats coating substantially entire peripheries thereof excluding the cutting portions, and the treatment portion being disposed at a tip end portion of the operation wire;
a connection terminal configured to apply a high frequency voltage to the cutting portions;
an operation portion installed in a base end portion of the flexible tube, and configured to open and close the gripping pieces by operating the treatment portion by performing an advance-retreat operation through the operation wire;
a holding frame that partially accommodates the treatment portion on an inner side between a pair of arm portions facing each other and holds both ends of the turning pivot; and
a spacer portion disposed between an inner surface of the arm portion and a counter-facing surface facing the inner surface of the arm portion in the treatment portion accommodated between the arm portions, and configured to separate the inner surface of the arm portion and the counter-facing surface of the treatment portionfrom each other.

12. The endoscopic high frequency treatment tool according to claim 11,
wherein the spacer portion is interposed between the counter-facing surface of the gripping piece accommodated between the arm portions and the inner surface of the arm portion, with no gap.

13. The endoscopic high frequency treatment tool according to claim 11 or 12,
wherein two of the spacer portions are disposed so as to face each other while interposing the treatment portion therebetween.

14. The endoscopic high frequency treatment tool according to any one of claims 11 to 13,
wherein the spacer portion is provided on a periphery of the turning pivot.

15. The endoscopic high frequency treatment tool according to any one of claims 11 to 14,
wherein the spacer portion is a plane plate-shaped body and has a hole penetrating the spacer portion in a thickness direction, and
wherein the turning pivot is inserted through the hole.

16. The endoscopic high frequency treatment tool according to claim 15,
wherein the spacer portion has a disk shape and is provided with the hole substantially at a center thereof.

17. The endoscopic high frequency treatment tool according to claim 16,
wherein an outer diameter of the spacer portion having a disk shape is equal to or greater than half a width size of a place penetrated by the turning pivot of the gripping pieces.

18. The endoscopic high frequency treatment tool according to any one of claims 11 to 17,
wherein the spacer portion is fixed to an outer peripheral surface of the gripping piece.

19. The endoscopic high frequency treatment tool according to claim 18,
wherein under an opening-closing operation of the pair of gripping pieces, the spacer portion is included inside of an outer edge of the arm portion viewed in a pivot center direction of the turning pivot.

20. The endoscopic high frequency treatment tool according to claim 18 or 19,
wherein the two spacer portions which are plane plate-shaped bodies are disposed so as to face each other while interposing the pair of gripping pieces therebetween, and
wherein a maximum size of the treatment portion in a pivot center direction of the turning pivot is smaller than a counter-facing width size of the two spacer portions facing each other.

21. The endoscopic high frequency treatment tool according to any one of claims 11 to 17,
wherein the spacer portion is fixed to the inner surface of the arm portion.

22. The endoscopic high frequency treatment tool according to claim 21,
wherein the gripping piece has a contact region which comes into contact with the spacer portion, and
wherein a trajectory of the contact region of the gripping piece configuring the pair of gripping pieces which are opened and closed so as to be in a released state and a closed state is included inside of an outer edge of the arm portion viewed in a pivot center direction of the turning pivot.

23. The endoscopic high frequency treatment tool according to any one of claims 11 to 22,
wherein the pair of gripping pieces is a pair of scissor pieces respectively having thin plate-shaped cutting portions.

24. The endoscopic high frequency treatment tool according to claim 23,
wherein the pair of scissor pieces individually has a tip end bent portion which is bent toward one side in the thickness direction.

25. The endoscopic high frequency treatment tool according to claim 24,
wherein the pair of scissor pieces individually has a base end bent portion which is bent toward the other side in the thickness direction, on a base end side beyond the tip end bent portion, and
wherein tip ends of the pair of scissor pieces are positioned on an extension line of the operation wire.

26. The endoscopic high frequency treatment tool according to any one of claims 11 to 25,
wherein the insulation coat comprises a silicon-based ceramic.

27. The endoscopic high frequency treatment tool according to any one of claims 11 to 26,
wherein the insulation coats respectively coating outer surfaces of the pair of gripping pieces are colored with colors different from each other.
